# EUROPEAN PATENT APPLICATION

(11) **EP 4 682 147 A1**
(43) Date of publication of application: **21.01.2026**
(21) Application number: 24774020.2
(22) Date of filing: 15.03.2024
(51) Int. Cl.: C07D 471/04, A61K 31/4375, A61P 9/00, A61P 9/12

(54) **PHARMACEUTICALLY ACCEPTABLE SALT AND CRYSTAL FORM OF 1,4-DIHYDRO-1,6-NAPHTHYRIDINE AMIDE COMPOUND AND PREPARATION METHOD THEREFOR**

(30) Priority: 17.03.2023 CN 202310260804; 17.03.2023 CN 202310260287
(71) Applicant: Jiangsu Hengrui Pharmaceuticals Co., Ltd., Lianyungang, Jiangsu 222047 (CN)
(72) Inventor: YAO, Jiaqi, Lianyungang, Jiangsu 222047 (CN); YANG, Junran, Lianyungang, Jiangsu 222047 (CN); DU, Zhenxing, Lianyungang, Jiangsu 222047 (CN); WANG, Jie, Lianyungang, Jiangsu 222047 (CN)
(74) Representative: dompatent
(86) International application number: PCT/CN2024/081878
(87) International publication number: WO 2024/193455

(57) **Abstract**

The present disclosure relates to a pharmaceutically acceptable salt (formula 1) and a crystal form of a 1,4-dihydro-1,6-naphthyridine amide compound, and a preparation method therefor. Specifically, provided in the present disclosure are a pharmaceutically acceptable salt and crystal form of (S)-4-(3-acetyl-5-ethoxy-2,8-dimethyl-1,4-dihydro-1,6-naphthyridine-4-yl)-3-(methoxy-*d₃*)benzonitrile, and a preparation method therefor. The corresponding crystal form has good stability and can be better used for clinical treatment.

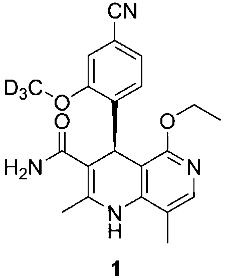

## Description

### The present application claims priority to:

CN202310260804.4, March 17, 2023;

CN202310260287.0, March 17, 2023.

### TECHNICAL FIELD

The present disclosure pertains to the technical field of pharmaceutics and relates to a pharmaceutically acceptable salt and a crystal form of a 1,4-dihydro-1,6-naphthyridine amide compound and a preparation method therefor.

### BACKGROUND

Mineralocorticoid receptors (MRs) are aldosterone-activated nuclear hormone receptors that regulate the expression of genes involved in electrolyte homeostasis and cardiovascular diseases. For example, an increase in circulating aldosterone increases blood pressure through its effect on natriuresis and meanwhile potentially affects the brain, the heart, and the vascular system. In addition, hyperaldosteronism is associated with many physiological processes leading to renal and cardiovascular diseases.

PCT/CN2022/119209 provides an MR antagonist, named (*S*)-4-(3-acetyl-5-ethoxy-2,8-dimethyl-1,4-dihydro-1,6-naphthyridin-4-yl)-3-(methoxy-*d₃*)benzonitrile, with the structure of formula 1,

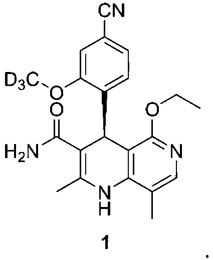

Salt formation can improve certain undesirable physicochemical or biological properties of drugs. It is of great significance to develop a salt having more excellent physicochemical or pharmaceutical properties relative to (*S*)-4-(3-acetyl-5-ethoxy-2,8-dimethyl-1,4-dihydro-1,6-naphthyridin-4-yl)-3-(methoxy-*d₃*)benzonitrile. In view of the importance of solid drug crystal forms and their stability in clinical treatment, intensive research on the polymorphs of pharmaceutically acceptable salts of the compound (*S*)-4-(3-acetyl-5-ethoxy-2,8-dimethyl-1,4-dihydro-1,6-naphthyridin-4-yl)-3-(methoxy-*d₃*)benzonitrile is also of great significance to develop drugs that are suitable for industrial production and have good biological activity.

### SUMMARY

The present disclosure provides a pharmaceutically acceptable salt of the compound of formula 1, (*S*)-4-(3-acetyl-5-ethoxy-2,8-dimethyl-1,4-dihydro-1,6-naphthyridin-4-yl)-3-(methoxy-*d₃*)benzonitrile, wherein the pharmaceutically acceptable salt is selected from the group consisting of hydrochloride, sulfate, phosphate, hydrobromide, mesylate, p-toluenesulfonate, tartrate, maleate, citrate, and malate,

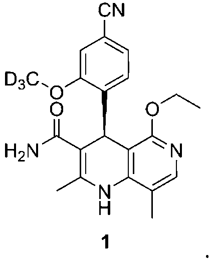

The present disclosure further provides a method for preparing a pharmaceutically acceptable salt of the compound of formula 1, the method comprising a step of reacting the compound of formula 1, (*S*)-4-(3-acetyl-5-ethoxy-2,8-dimethyl-1,4-dihydro-1,6-naphthyridin-4-yl)-3-(methoxy-*d₃*)benzonitrile, with an acid, wherein the acid is selected from the group consisting of hydrochloric acid, sulfuric acid, phosphoric acid, hydrobromic acid, methanesulfonic acid, p-toluenesulfonic acid, tartaric acid, maleic acid, citric acid, and malic acid.

The solvent used for salt formation in the present disclosure is selected from the group consisting of, but is not limited to, acetone/water, acetonitrile/water, isopropanol, isopropyl acetate, 4-methyl-2-pentanone, ethanol/dichloromethane, and n-heptane.

Further, in an optional embodiment, the method for preparing the aforementioned pharmaceutically acceptable salt further comprises such a step as crystallization, filtration, washing, or drying.

In an optional embodiment, the chemical ratio of the compound of formula 1 to the acid is 3:1-1:3, including but not limited to, 3:1, 2:1, 1:1, 1:2, and 1:3.

In another embodiment, the chemical ratio of the compound of formula 1 to the acid is 2:1-1:2.

In an optional embodiment, the chemical ratio of the compound of formula 1 to hydrochloric acid is 1:1.

In an optional embodiment, the chemical ratio of the compound of formula 1 to sulfuric acid is 1:1. In an optional embodiment, the chemical ratio of the compound of formula 1 to phosphoric acid is 1:1. In an optional embodiment, the chemical ratio of the compound of formula 1 to hydrobromic acid is 1:1.

In an optional embodiment, the chemical ratio of the compound of formula 1 to methanesulfonic acid is 1:1.

In an optional embodiment, the chemical ratio of the compound of formula 1 to p-toluenesulfonic acid is 1:1.

In an optional embodiment, the chemical ratio of the compound of formula 1 to tartaric acid is 1:1. In an optional embodiment, the chemical ratio of the compound of formula 1 to tartaric acid is 2:1. In an optional embodiment, the chemical ratio of the compound of formula 1 to maleic acid is 1:1.

In an optional embodiment, the chemical ratio of the compound of formula 1 to malic acid is 1:1.

In another aspect, the present disclosure provides a crystal form I of mesylate of the compound of formula 1, wherein an X-ray powder diffraction pattern expressed in terms of 2*θ* angles, which are diffraction angles, of the crystal form has characteristic peaks at 7.401, 8.635, 9.630, 17.457, and 26.350.

In some embodiments, the X-ray powder diffraction pattern expressed in terms of 2*θ* angles, which are diffraction angles, of the crystal form I of mesylate of the compound of formula 1 has characteristic peaks at 7.401, 8.635, 9.630, 14.895, 17.457, 19.014, 26.350, and 27.833.

In some embodiments, the X-ray powder diffraction pattern expressed in terms of 2*θ* angles, which are diffraction angles, of the crystal form I of mesylate of the compound of formula 1 has characteristic peaks at 7.401, 8.635, 9.630, 14.895, 15.590, 17.457, 19.014, 19.529, 22.735, 26.350, and 27.833.

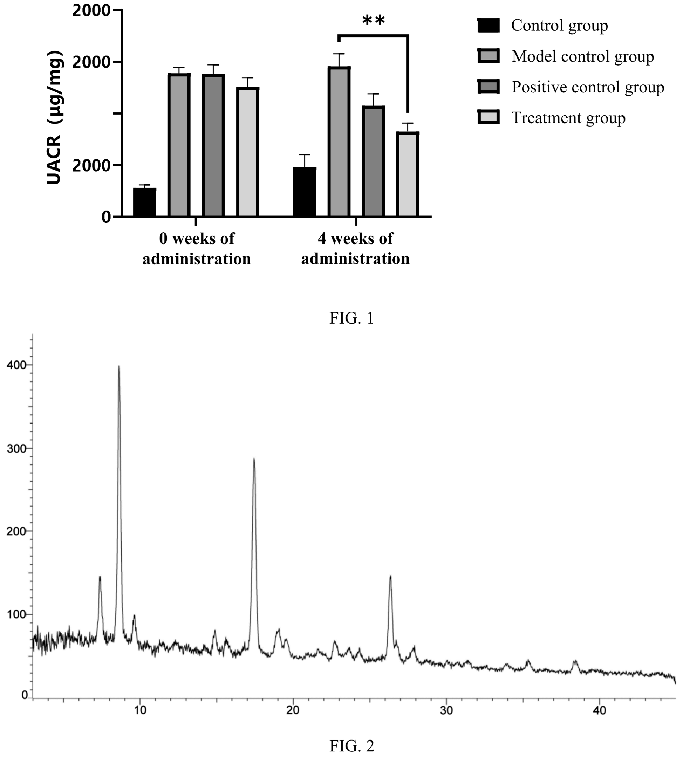

In some embodiments, the X-ray powder diffraction pattern expressed in terms of 2*θ* angles, which are diffraction angles, of the crystal form I of mesylate of the compound of formula 1 is shown in FIG. 2. The present disclosure further provides a method for preparing the crystal form I of mesylate of the compound of formula 1, the method comprising:
method I: dissolving the compound of formula 1 in 90% acetone/water, adding an aqueous methanesulfonic acid solution, and stirring;
method II: adding the compound of formula 1 to a solvent (1), adding a solution of methanesulfonic acid in ethanol, and slurrying for crystallization, wherein the solvent (1) is selected from the group consisting of isopropanol, isopropyl acetate, and 4-methyl-2-pentanone;
method III: dissolving the compound of formula 1 in acetonitrile/water (v/v = 1:1), adding a solution of methanesulfonic acid in ethanol, completely dissolving the mixture, and volatilizing for crystallization.

In another aspect, the present disclosure provides a crystal form α of maleate of the compound of formula 1, wherein an X-ray powder diffraction pattern expressed in terms of 2*θ* angles, which are diffraction angles, of the crystal form has characteristic peaks at 7.147, 8.562, 17.254, 20.869, and 26.061.

In some embodiments, the X-ray powder diffraction pattern expressed in terms of 2*θ* angles, which are diffraction angles, of the crystal form α of maleate of the compound of formula 1 has characteristic peaks at 7.147, 8.562, 11.015, 12.787, 14.399, 17.254, 20.869, 22.478, and 26.061.

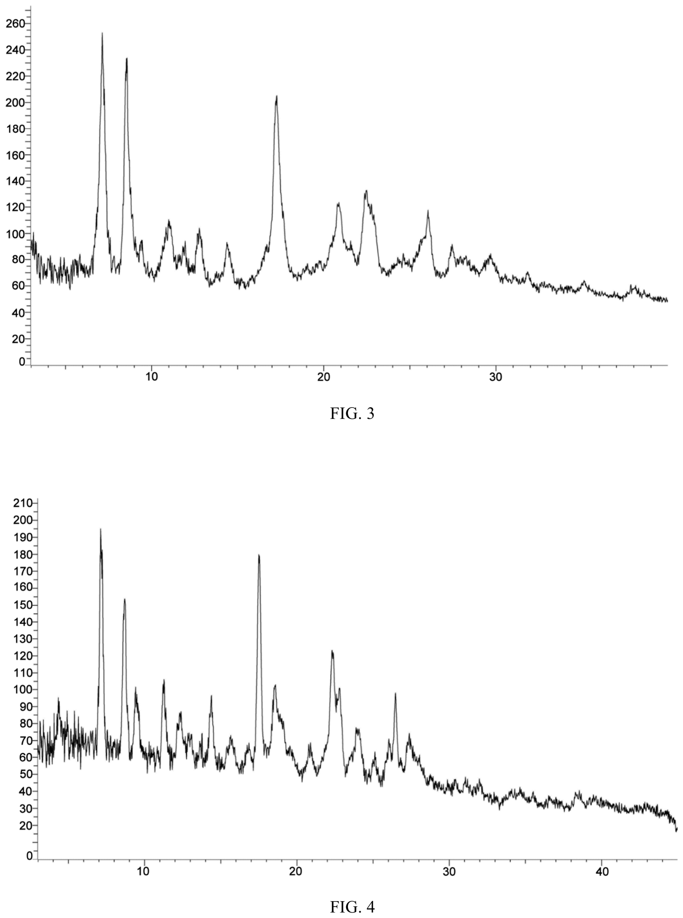

In some embodiments, the X-ray powder diffraction pattern expressed in terms of 2*θ* angles, which are diffraction angles, of the crystal form α of maleate of the compound of formula 1 is shown in FIG. 3. The present disclosure further provides a method for preparing the crystal form α of maleate of the compound of formula 1, the method comprising:
method I: adding the compound of formula 1 to a solvent (2), adding maleic acid, and slurrying for crystallization, wherein the solvent (2) is selected from the group consisting of isopropanol, isopropyl acetate, 4-methyl-2-pentanone, and 90% acetonitrile/water;
method II: dissolving the compound of formula 1 in ethanol/dichloromethane (v/v = 1:1), adding an aqueous maleic acid solution, and then adding n-heptane for crystallization.

In another aspect, the present disclosure provides a crystal form a of sulfate of the compound of formula 1, wherein an X-ray powder diffraction pattern expressed in terms of 2*θ* angles, which are diffraction angles, of the crystal form has characteristic peaks at 7.130, 8.699, 17.528, 22.332, and 26.473.

In some embodiments, the X-ray powder diffraction pattern expressed in terms of 2*θ* angles, which are diffraction angles, of the crystal form a of sulfate of the compound of formula 1 has characteristic peaks at 7.130, 8.699, 9.465, 11.261, 17.528, 18.564, 22.332, and 26.473.

In some embodiments, the X-ray powder diffraction pattern expressed in terms of 2*θ* angles, which are diffraction angles, of the crystal form a of sulfate of the compound of formula 1 has characteristic peaks at 7.130, 8.699, 9.465, 11.261, 14.388, 15.667, 17.528, 18.564, 22.332, 22.798, 26.473, and 27.331.

In some embodiments, the X-ray powder diffraction pattern expressed in terms of 2*θ* angles, which are diffraction angles, of the crystal form a of sulfate of the compound of formula 1 is shown in FIG. 4. The present disclosure further provides a method for preparing the crystal form a of sulfate of the compound of formula 1, the method comprising:
method I: dissolving the compound of formula 1 in 90% acetone/water, adding an aqueous sulfuric acid solution, and stirring;
method II: adding the compound of formula 1 to a solvent (3), adding a solution of sulfuric acid in ethanol, and slurrying for crystallization, wherein the solvent (3) is selected from the group consisting of isopropanol, isopropyl acetate, and 4-methyl-2-pentanone;
method III: dissolving the compound of formula 1 in acetonitrile/water (v/v = 1:1), adding a solution of sulfuric acid in ethanol, completely dissolving the mixture, and volatilizing for crystallization.

In another aspect, the present disclosure provides a crystal form a of tartrate of the compound of formula 1, wherein an X-ray powder diffraction pattern expressed in terms of 2*θ* angles, which are diffraction angles, of the crystal form has characteristic peaks at 7.474, 10.546, 19.022, 20.714, 22.866, and 25.520.

In some embodiments, the X-ray powder diffraction pattern expressed in terms of 2*θ* angles, which are diffraction angles, of the crystal form a of tartrate of the compound of formula 1 has characteristic peaks at 6.838, 7.474, 8.453, 10.546, 17.246, 19.022, 20.714, 22.866, 25.520, and 27.173.

In some embodiments, the X-ray powder diffraction pattern expressed in terms of 2*θ* angles, which are diffraction angles, of the crystal form a of tartrate of the compound of formula 1 has characteristic peaks at 6.838, 7.474, 8.453, 10.546, 17.246, 19.022, 20.714, 22.866, 25.520, 26.461, 27.173, and 27.835.

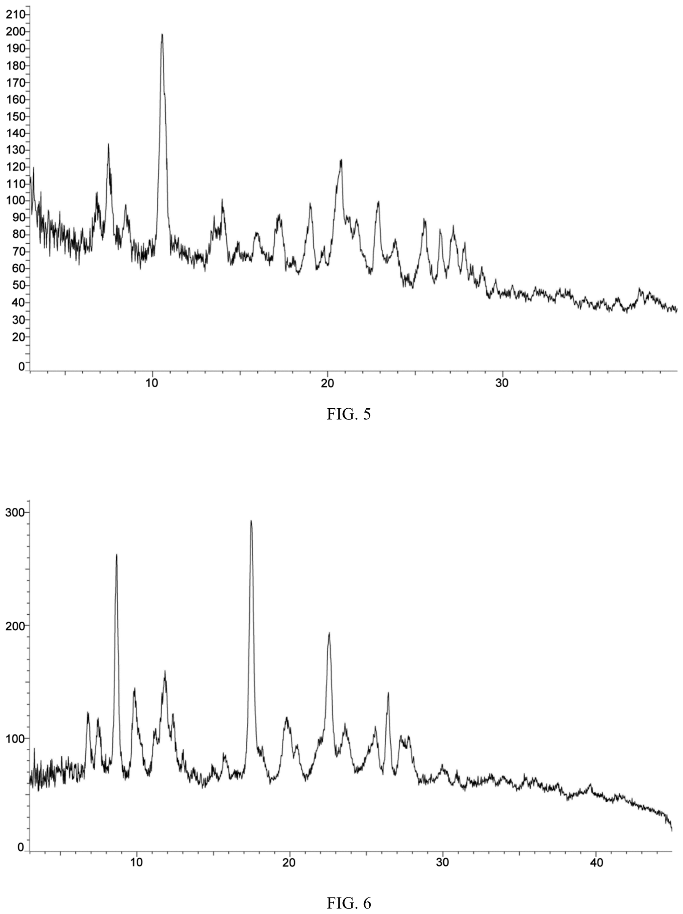

In some embodiments, the X-ray powder diffraction pattern expressed in terms of 2*θ* angles, which are diffraction angles, of the crystal form a of tartrate of the compound of formula 1 is shown in FIG. 5. The present disclosure further provides a method for preparing the crystal form a of tartrate of the compound of formula 1, the method comprising: adding the compound of formula 1 to isopropanol, adding tartaric acid, and slurrying for crystallization.

In another aspect, the present disclosure provides a crystal form b of tartrate of the compound of formula 1, wherein an X-ray powder diffraction pattern expressed in terms of 2*θ* angles, which are diffraction angles, of the crystal form has characteristic peaks at 8.674, 9.822, 11.826, 17.482, 22.566, and 26.431.

In some embodiments, the X-ray powder diffraction pattern expressed in terms of 2*θ* angles, which are diffraction angles, of the crystal form b of tartrate of the compound of formula 1 has characteristic peaks at 6.823, 7.456, 8.674, 9.822, 11.826, 17.482, 19.798, 22.566, and 26.431.

In some embodiments, the X-ray powder diffraction pattern expressed in terms of 2*θ* angles, which are diffraction angles, of the crystal form b of tartrate of the compound of formula 1 has characteristic peaks at 6.823, 7.456, 8.674, 9.822, 11.826, 17.482, 19.798, 22.566, 23.584, 25.602, 26.431, 27.215, and 27.755.

In some embodiments, the X-ray powder diffraction pattern expressed in terms of 2*θ* angles, which are diffraction angles, of the crystal form b of tartrate of the compound of formula 1 is shown in FIG. 6. The present disclosure further provides a method for preparing the crystal form b of tartrate of the compound of formula 1, the method comprising:
method I: adding the compound of formula 1 to 90% acetonitrile/water, adding tartaric acid, and slurrying for crystallization;
method II: adding the compound of formula 1 to ethanol/dichloromethane (v/v = 1:1), adding an aqueous tartaric acid solution, completely dissolving the mixture, adding n-heptane, and slurrying for crystallization.

In another aspect, the present disclosure provides a crystal form I of hydrochloride of the compound of formula 1, wherein an X-ray powder diffraction pattern expressed in terms of 2*θ* angles, which are diffraction angles, of the crystal form has characteristic peaks at 5.266, 7.585, 10.560, 15.645, 22.452, and 27.841.

In some embodiments, the X-ray powder diffraction pattern expressed in terms of 2*θ* angles, which are diffraction angles, of the crystal form I of hydrochloride of the compound of formula 1 has characteristic peaks at 5.266, 7.585, 10.560, 11.394, 14.873, 15.645, 22.452, 23.008, and 27.841.

In some embodiments, the X-ray powder diffraction pattern expressed in terms of 2*θ* angles, which are diffraction angles, of the crystal form I of hydrochloride of the compound of formula 1 has characteristic peaks at 5.266, 7.585, 9.893, 10.560, 11.394, 14.873, 15.645, 17.447, 22.452, 23.008, 27.841, and 30.150.

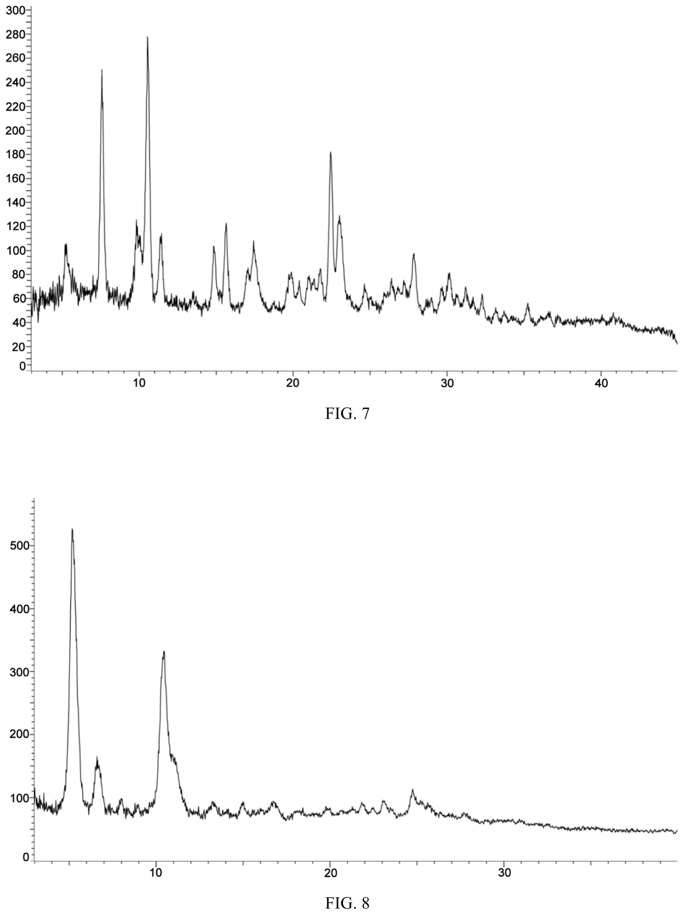

In some embodiments, the X-ray powder diffraction pattern expressed in terms of 2*θ* angles, which are diffraction angles, of the crystal form I of hydrochloride of the compound of formula 1 is shown in FIG. 7.

The present disclosure further provides a method for preparing the crystal form I of hydrochloride of the compound of formula 1, the method comprising:
method I: adding the compound of formula 1 to a solvent (4), adding a solution of hydrochloric acid in ethanol, and slurrying for crystallization, wherein the solvent (4) is selected from the group consisting of isopropanol, isopropyl acetate, and 4-methyl-2-pentanone;
method II: dissolving the compound of formula 1 in acetonitrile/water (v/v = 1:1), adding a solution of hydrochloric acid in ethanol, completely dissolving the mixture, and volatilizing for crystallization. In another aspect, the present disclosure provides a crystal form a of p-toluenesulfonate of the compound of formula 1, wherein an X-ray powder diffraction pattern expressed in terms of 2*θ* angles, which are diffraction angles, of the crystal form has characteristic peaks at 5.160, 6.628, 10.421, 13.342, and 24.762.

In some embodiments, the X-ray powder diffraction pattern expressed in terms of 2*θ* angles, which are diffraction angles, of the crystal form a of p-toluenesulfonate of the compound of formula 1 has characteristic peaks at 5.160, 6.628, 10.421, 10.934, 13.342, 16.744, 23.072, and 24.762.

In some embodiments, the X-ray powder diffraction pattern expressed in terms of 2*θ* angles, which are diffraction angles, of the crystal form a of p-toluenesulfonate of the compound of formula 1 is shown in FIG. 8.

The present disclosure further provides a method for preparing the crystal form a of p-toluenesulfonate of the compound of formula 1, the method comprising: adding the compound of formula 1 to isopropanol, adding p-toluenesulfonic acid, and slurrying for crystallization.

In another aspect, the present disclosure provides a crystal form b of p-toluenesulfonate of the compound of formula 1, wherein an X-ray powder diffraction pattern expressed in terms of 2*θ* angles, which are diffraction angles, of the crystal form has characteristic peaks at 8.410, 9.493, 13.727, 22.196, and 26.312.

In some embodiments, the X-ray powder diffraction pattern expressed in terms of 2*θ* angles, which are diffraction angles, of the crystal form b of p-toluenesulfonate of the compound of formula 1 has characteristic peaks at 8.410, 9.493, 11.535, 13.727, 17.760, 22.196, and 26.312.

In some embodiments, the X-ray powder diffraction pattern expressed in terms of 2*θ* angles, which are diffraction angles, of the crystal form b of p-toluenesulfonate of the compound of formula 1 has characteristic peaks at 8.410, 9.493, 11.535, 13.727, 17.760, 22.196, 24.857, 25.144, and 26.312.

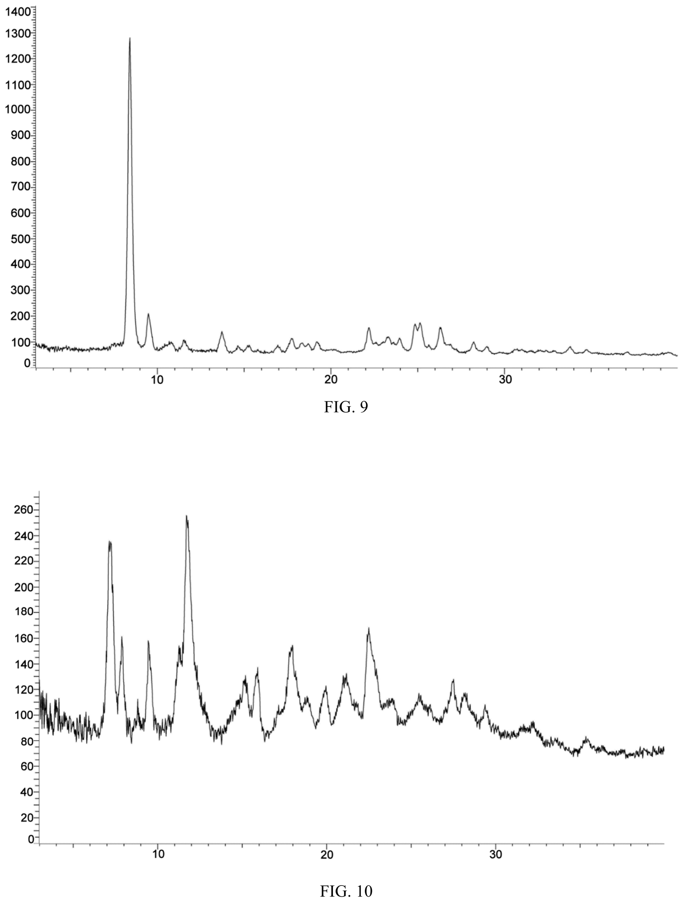

In some embodiments, the X-ray powder diffraction pattern expressed in terms of 2*θ* angles, which are diffraction angles, of the crystal form b of p-toluenesulfonate of the compound of formula 1 is shown in FIG. 9.

The present disclosure further provides a method for preparing the crystal form b of p-toluenesulfonate of the compound of formula 1, the method comprising: adding the compound of formula 1 to 90% acetonitrile/water, adding p-toluenesulfonic acid, and slurrying for crystallization.

In another aspect, the present disclosure provides a crystal form a of citrate of the compound of formula 1, wherein an X-ray powder diffraction pattern expressed in terms of 2*θ* angles, which are diffraction angles, of the crystal form has characteristic peaks at 7.218, 9.479, 11.757, 17.927, and 22.498.

In some embodiments, the X-ray powder diffraction pattern expressed in terms of 2*θ* angles, which are diffraction angles, of the crystal form a of citrate of the compound of formula 1 has characteristic peaks at 7.218, 7.899, 9.479, 11.307, 11.757, 15.875, 17.927, 19.942, and 22.498.

In some embodiments, the X-ray powder diffraction pattern expressed in terms of 2*θ* angles, which are diffraction angles, of the crystal form a of citrate of the compound of formula 1 has characteristic peaks at 7.218, 7.899, 9.479, 11.307, 11.757, 15.875, 17.927, 19.942, 21.133, 22.498, and 27.461.

In some embodiments, the X-ray powder diffraction pattern expressed in terms of 2*θ* angles, which are diffraction angles, of the crystal form a of citrate of the compound of formula 1 is shown in FIG. 10. The present disclosure further provides a method for preparing the crystal form a of citrate of the compound of formula 1, the method comprising: adding the compound of formula 1 and citric acid to 90% acetonitrile/water, completely dissolving the mixture, and volatilizing for crystallization.

In another aspect, the present disclosure provides a crystal form I of malate of the compound of formula 1, wherein an X-ray powder diffraction pattern expressed in terms of 2*θ* angles, which are diffraction angles, of the crystal form has characteristic peaks at 7.194, 10.779, 15.263, 21.782, and 29.155.

In some embodiments, the X-ray powder diffraction pattern expressed in terms of 2*θ* angles, which are diffraction angles, of the crystal form I of malate of the compound of formula 1 has characteristic peaks at 7.194, 10.779, 11.339, 12.996, 15.263, 21.782, and 29.155.

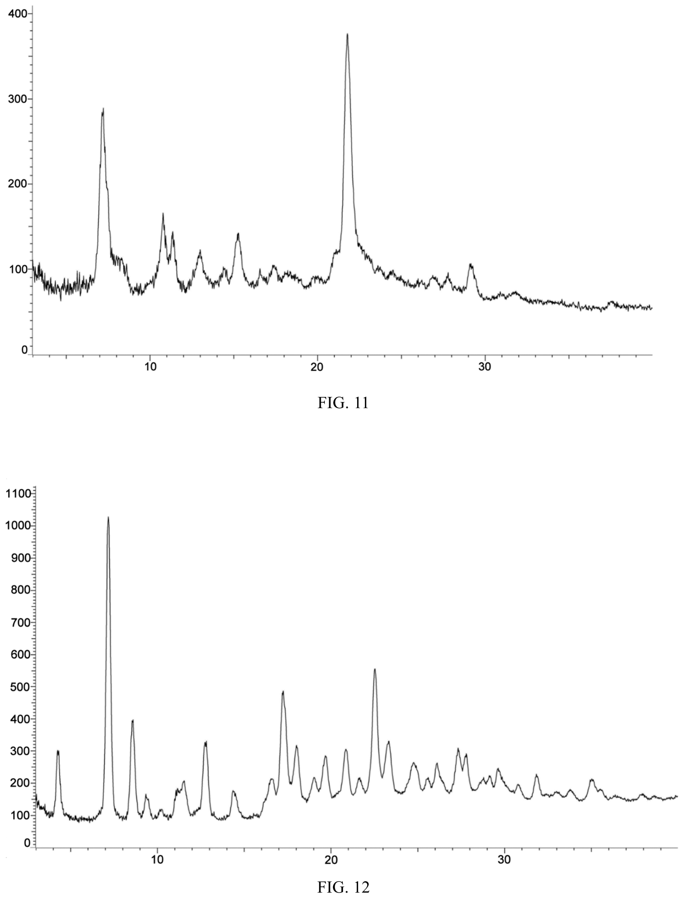

In some embodiments, the X-ray powder diffraction pattern expressed in terms of 2*θ* angles, which are diffraction angles, of the crystal form I of malate of the compound of formula 1 is shown in FIG. 11. The present disclosure further provides a method for preparing the crystal form I of malate of the compound of formula 1, the method comprising: adding the compound of formula 1 to a solvent (5), adding malic acid, and slurrying for crystallization, wherein the solvent (5) is selected from the group consisting of isopropyl acetate and 4-methyl-2-pentanone.

In another aspect, the present disclosure provides a crystal form II of malate of the compound of formula 1, wherein an X-ray powder diffraction pattern expressed in terms of 2*θ* angles, which are diffraction angles, of the crystal form has characteristic peaks at 4.264, 7.177, 8.583, 12.752, 17.248, and 22.530.

In some embodiments, the X-ray powder diffraction pattern expressed in terms of 2*θ* angles, which are diffraction angles, of the crystal form II of malate of the compound of formula 1 has characteristic peaks at 4.264, 7.177, 8.583, 12.752, 14.419, 17.248, 18.023, 20.863, 22.530, and 23.333.

In some embodiments, the X-ray powder diffraction pattern expressed in terms of 2*θ* angles, which are diffraction angles, of the crystal form II of malate of the compound of formula 1 has characteristic peaks at 4.264, 7.177, 8.583, 12.752, 14.419, 17.248, 18.023, 19.696, 20.863, 22.530, 23.333, 24.766, 27.328, and 27.757.

In some embodiments, the X-ray powder diffraction pattern expressed in terms of 2*θ* angles, which are diffraction angles, of the crystal form II of malate of the compound of formula 1 is shown in FIG. 12. The present disclosure further provides a method for preparing the crystal form II of malate of the compound of formula 1, the method comprising: adding the compound of formula 1 and malic acid to 90% acetonitrile/water, completely dissolving the mixture, and volatilizing for crystallization.

In another aspect, the present disclosure provides a crystal form III of malate of the compound of formula 1, wherein an X-ray powder diffraction pattern expressed in terms of 2*θ* angles, which are diffraction angles, of the crystal form has characteristic peaks at 6.847, 8.609, 11.179, 17.410, and 22.494.

In some embodiments, the X-ray powder diffraction pattern expressed in terms of 2*θ* angles, which are diffraction angles, of the crystal form III of malate of the compound of formula 1 has characteristic peaks at 6.847, 8.609, 9.815, 11.179, 17.410, and 22.494.

In some embodiments, the X-ray powder diffraction pattern expressed in terms of 2*θ* angles, which are diffraction angles, of the crystal form III of malate of the compound of formula 1 has characteristic peaks at 6.847, 8.609, 9.815, 11.179, 17.410, 19.023, 20.927, 22.494, and 27.351.

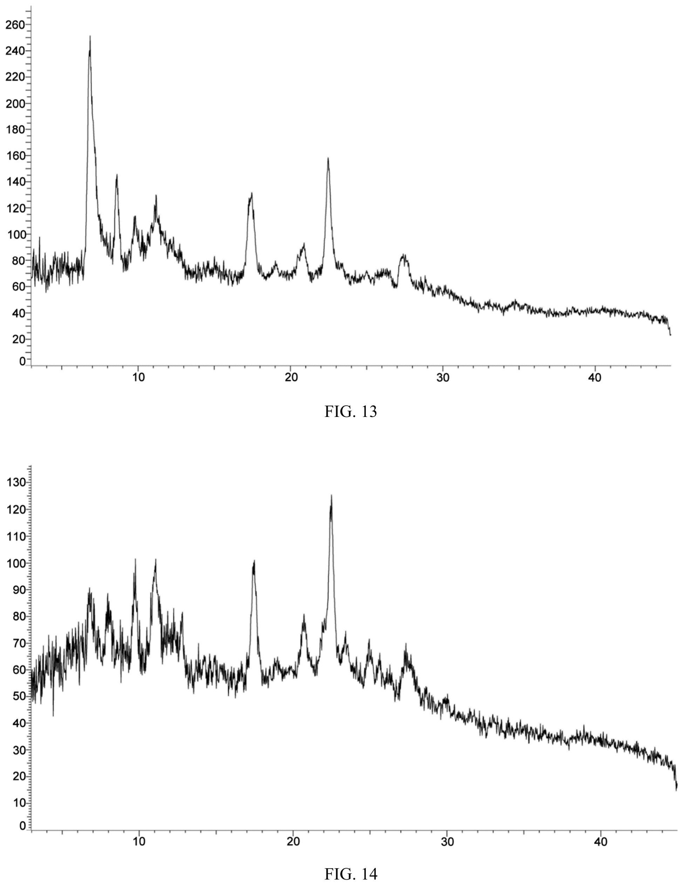

In some embodiments, the X-ray powder diffraction pattern expressed in terms of 2*θ* angles, which are diffraction angles, of the crystal form III of malate of the compound of formula 1 is shown in FIG. 13. The present disclosure further provides a method for preparing the crystal form III of malate of the compound of formula 1, the method comprising: heating the crystal form II of malate of the compound of formula 1 to 110 °C.

In another aspect, the present disclosure provides a crystal form α of phosphate of the compound of formula 1, wherein an X-ray powder diffraction pattern expressed in terms of 2*θ* angles, which are diffraction angles, of the crystal form has characteristic peaks at 8.021, 9.756, 11.072, 17.455, and 22.489.

In some embodiments, the X-ray powder diffraction pattern expressed in terms of 2*θ* angles, which are diffraction angles, of the crystal form α of phosphate of the compound of formula 1 has characteristic peaks at 6.785, 8.021, 9.756, 11.072, 17.455, 20.688, 22.489, and 27.369.

In some embodiments, the X-ray powder diffraction pattern expressed in terms of 2*θ* angles, which are diffraction angles, of the crystal form α of phosphate of the compound of formula 1 is shown in FIG. 14.

The present disclosure further provides a method for preparing the crystal form α of phosphate of the compound of formula 1, the method comprising:
method I: adding the compound of formula 1 to a solvent (6), adding a solution of phosphoric acid in ethanol, and slurrying for crystallization, wherein the solvent (6) is selected from the group consisting of isopropyl acetate and 4-methyl-2-pentanone;
method II: adding the compound of formula 1 to acetonitrile/water (v/v = 1:1), adding a solution of phosphoric acid in ethanol, completely dissolving the mixture, and volatilizing for crystallization.

In another aspect, the present disclosure provides a crystal form I of hydrobromide of the compound of formula 1, wherein an X-ray powder diffraction pattern expressed in terms of 2*θ* angles, which are diffraction angles, of the crystal form has characteristic peaks at 5.089, 7.489, 10.392, 22.377, and 29.592.

In some embodiments, the X-ray powder diffraction pattern expressed in terms of 2*θ* angles, which are diffraction angles, of the crystal form I of hydrobromide of the compound of formula 1 has characteristic peaks at 5.089, 7.489, 10.392, 11.377, 14.615, 19.581, 22.377, and 29.592.

In some embodiments, the X-ray powder diffraction pattern expressed in terms of 2*θ* angles, which are diffraction angles, of the crystal form I of hydrobromide of the compound of formula 1 has characteristic peaks at 5.089, 7.489, 10.392, 11.377, 14.615, 15.112, 19.581, 22.377, 27.675, 29.592, and 31.726.

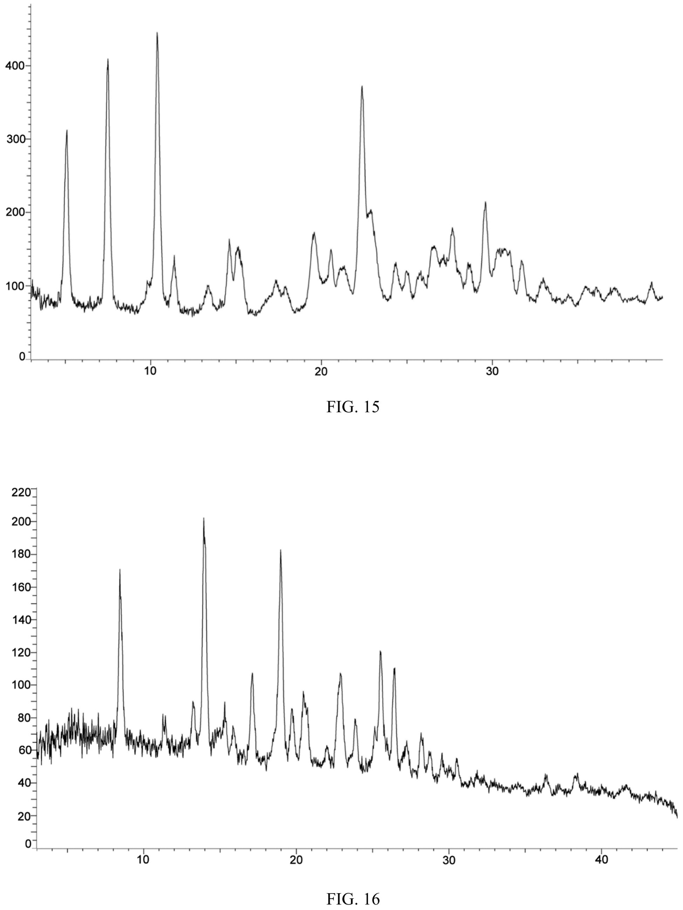

In some embodiments, the X-ray powder diffraction pattern expressed in terms of 2*θ* angles, which are diffraction angles, of the crystal form I of hydrobromide of the compound of formula 1 is shown in FIG. 15.

The present disclosure further provides a method for preparing the crystal form I of hydrobromide of the compound of formula 1, the method comprising:
method I: adding the compound of formula 1 to a solvent (7), adding a solution of hydrobromic acid in ethanol, and slurrying for crystallization, wherein the solvent (7) is selected from the group consisting of isopropanol, isopropyl acetate, and 4-methyl-2-pentanone;
method II: adding the compound of formula 1 to methanol/dichloromethane (v/v = 1:1), adding a solution of hydrobromic acid in ethanol, then adding ethyl acetate, and stirring for crystallization; method III: adding the compound of formula 1 to 90% acetonitrile/water, adding a solution of hydrobromic acid in ethanol, completely dissolving the mixture, and volatilizing for crystallization. In another aspect, the present disclosure provides a crystal form A of the compound of formula 1, wherein an X-ray powder diffraction pattern expressed in terms of 2*θ* angles, which are diffraction angles, of the crystal form has characteristic peaks at 8.459, 13.964, 18.987, 22.914, and 25.530.

In some embodiments, the X-ray powder diffraction pattern expressed in terms of 2*θ* angles, which are diffraction angles, of the crystal form A of the compound of formula 1 has characteristic peaks at 8.459, 13.964, 17.119, 18.987, 20.481, 22.914, 25.530, and 26.428.

In some embodiments, the X-ray powder diffraction pattern expressed in terms of 2*θ* angles, which are diffraction angles, of the crystal form A of the compound of formula 1 has characteristic peaks at 8.459, 13.248, 13.964, 17.119, 18.987, 19.709, 20.481, 22.914, 23.874, 25.530, and 26.428.

In some embodiments, the X-ray powder diffraction pattern expressed in terms of 2*θ* angles, which are diffraction angles, of the crystal form A of the compound of formula 1 is shown in FIG. 16.

The present disclosure further provides a method for preparing the crystal form A of the compound of formula 1, wherein the method is selected from the group consisting of:
method I: adding the compound of formula 1 to a solvent I, and slurrying for crystallization, wherein the solvent I is selected from the group consisting of one or more of water, isopropanol, isopropyl acetate, methyl tert-butyl ether, isopropyl ether, toluene, n-heptane, ethyl acetate, 4-methyl-2-pentanone, and acetonitrile; and
method II: dissolving the compound of formula 1 in a solvent II, and adding a solvent III for crystallization, wherein the solvent II is selected from the group consisting of one or more of dimethyl sulfoxide, methanol, dichloromethane, acetone, and acetonitrile, and the solvent III is selected from the group consisting of one or more of water, isopropyl ether, isopropyl acetate, n-heptane, and methyl tert-butyl ether.

The present disclosure further provides a pharmaceutical composition comprising the aforementioned crystal form A, crystal form I of mesylate, crystal form α of maleate, crystal form a of sulfate, crystal form a of tartrate, crystal form b of tartrate, crystal form I of hydrochloride, crystal form a of p-toluenesulfonate, crystal form b of p-toluenesulfonate, crystal form a of citrate, crystal form I of malate, crystal form II of malate, crystal form III of malate, crystal form α of phosphate, and crystal form I of hydrobromide of the compound of formula 1, or a pharmaceutically acceptable salt of the compound of formula 1, optionally a pharmaceutically acceptable auxiliary material selected from the group consisting of pharmaceutically acceptable excipients.

The present disclosure further provides a pharmaceutical composition prepared from the aforementioned crystal form A, crystal form I of mesylate, crystal form α of maleate, crystal form a of sulfate, crystal form a of tartrate, crystal form b of tartrate, crystal form I of hydrochloride, crystal form a of p-toluenesulfonate, crystal form b of p-toluenesulfonate, crystal form a of citrate, crystal form I of malate, crystal form II of malate, crystal form III of malate, crystal form α of phosphate, and crystal form I of hydrobromide of the compound of formula 1, or a pharmaceutically acceptable salt of the compound of formula 1, and optionally a pharmaceutically acceptable excipient.

The present disclosure further provides a method for preparing a pharmaceutical composition, the method comprising a step of mixing the aforementioned crystal form A, crystal form I of mesylate, crystal form α of maleate, crystal form a of sulfate, crystal form a of tartrate, crystal form b of tartrate, crystal form I of hydrochloride, crystal form a of p-toluenesulfonate, crystal form b of p-toluenesulfonate, crystal form a of citrate, crystal form I of malate, crystal form II of malate, crystal form III of malate, crystal form α of phosphate, and crystal form I of hydrobromide of the compound of formula 1, or a pharmaceutically acceptable salt of the compound of formula 1 with a pharmaceutically acceptable excipient.

The present disclosure further provides use of the aforementioned crystal form A, crystal form I of mesylate, crystal form α of maleate, crystal form a of sulfate, crystal form a of tartrate, crystal form b of tartrate, crystal form I of hydrochloride, crystal form a of p-toluenesulfonate, crystal form b of p-toluenesulfonate, crystal form a of citrate, crystal form I of malate, crystal form II of malate, crystal form III of malate, crystal form α of phosphate, and crystal form I of hydrobromide of the compound of formula 1, or a pharmaceutically acceptable salt of the compound of formula 1, or the aforementioned composition in the manufacture of a medicament for preventing and/or treating a disease or disorder associated with a mineralocorticoid.

The present disclosure further provides use of the aforementioned crystal form A, crystal form I of mesylate, crystal form α of maleate, crystal form a of sulfate, crystal form a of tartrate, crystal form b of tartrate, crystal form I of hydrochloride, crystal form a of p-toluenesulfonate, crystal form b of p-toluenesulfonate, crystal form a of citrate, crystal form I of malate, crystal form II of malate, crystal form III of malate, crystal form α of phosphate, and crystal form I of hydrobromide of the compound of formula 1, or a pharmaceutically acceptable salt of the compound of formula 1, or the aforementioned composition in the manufacture of a medicament for preventing and/or treating hyperaldosteronism, hypertension, and heart failure.

As used herein, the "2*θ* or 2*θ* angle" refers to a diffraction angle; *θ* refers to the Bragg angle in ° or degrees; the 2*θ* of each characteristic peak has a margin of error of ±0.20 (including the case that a number having more than 1 decimal place is rounded), specifically -0.20, -0.19, -0.18, -0.17, -0.16, - 0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, or 0.20.

In the present disclosure, numerical values such as the content of related substances are data obtained by measurement and calculation, and there is inevitably a certain degree of error. In general, ±10% is a reasonable margin of error. There is a certain degree of error change depending on the context where it is used, and the error change is no more than ±10% and may be ±9%, ±8%, ±7%, ±6%, ±5%, ±4%, ±3%, ±2%, or ±1%, preferably ±5%.

The starting material used in the methods for preparing the crystal forms of the present disclosure may be any form of the compound, and the specific forms include, but are not limited to: an amorphous form, any crystal form, a hydrate, a solvate, and the like.

In the present disclosure, the drying temperature is generally 25 °C-100 °C, preferably 40 °C-70 °C, and the drying may be performed at an atmospheric pressure or reduced pressure.

Methods for the crystallization described in the present disclosure include room-temperature crystallization, cooling crystallization, crystallization by volatilizing the solvent, crystallization induced by adding a seed crystal, and the like, wherein the cooling temperature is selected from the group consisting of 65 °C or lower, preferably from the group consisting of -10 °C to 60 °C, and the crystallization process may involve stirring.

As used herein, the "differential scanning calorimetry" or "DSC" refers to the measurement of the temperature difference and heat flow difference between a sample and a reference substance during a ramping or thermostatic process to characterize all the physical changes and chemical changes related to the thermal effect, and to acquire phase change information about the sample.

According to the description of hygroscopicity characteristics and the definitions of hygroscopic weight gains in "General Rule 9103, Guidelines for Hygroscopicity" in the Chinese Pharmacopoeia, Volume IV, 2015 Edition,
deliquescent: sufficient water is absorbed to form a liquid;
extremely hygroscopic: the hygroscopic weight gain is not less than 15%;
hygroscopic: the hygroscopic weight gain is less than 15% but not less than 2%;
slightly hygroscopic: the hygroscopic weight gain is less than 2% but not less than 0.2%; nonhygroscopic or practically nonhygroscopic: the hygroscopic weight gain is less than 0.2%.

As used herein, the "excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, or emulsifier that has been approved by the U.S. Food and Drug Administration as acceptable for use in humans or livestock animals.

### BRIEF DESCRIPTION OF THE DRAWINGS

FIG. 1 shows the urine albumin-creatinine ratios (UACRs) of groups of mice.
FIG. 2 shows an XRPD pattern of the crystal form I of mesylate of compound 1.
FIG. 3 shows an XRPD pattern of the crystal form α of maleate of compound 1.
FIG. 4 shows an XRPD pattern of the crystal form a of sulfate of compound 1.
FIG. 5 shows an XRPD pattern of the crystal form a of tartrate of compound 1.
FIG. 6 shows an XRPD pattern of the crystal form b of tartrate of compound 1.
FIG. 7 shows an XRPD pattern of the crystal form I of hydrochloride of compound 1.
FIG. 8 shows an XRPD pattern of the crystal form a of p-toluenesulfonate of compound 1.
FIG. 9 shows an XRPD pattern of the crystal form b of p-toluenesulfonate of compound 1.
FIG. 10 shows an XRPD pattern of the crystal form a of citrate of compound 1.
FIG. 11 shows an XRPD pattern of the crystal form I of malate of compound 1.
FIG. 12 shows an XRPD pattern of the crystal form II of malate of compound 1.
FIG. 13 shows an XRPD pattern of the crystal form III of malate of compound 1.
FIG. 14 shows an XRPD pattern of the crystal form α of phosphate of compound 1.
FIG. 15 shows an XRPD pattern of the crystal form I of hydrobromide of compound 1.
FIG. 16 shows an XRPD pattern of the crystal form A of compound 1.
FIG. 17 shows an XRPD pattern of the amorphous form of compound 1.

### DETAILED DESCRIPTION

The present disclosure is explained below in greater detail with reference to examples or experimental examples. The examples or experimental examples in the present disclosure are only illustrative of the technical solutions in the present disclosure and do not limit the spirit and scope of the present disclosure.

Experimental methods without specific conditions indicated in the examples of the present disclosure were generally conducted under conventional conditions, or conditions recommended by the manufacturers of the starting materials or commercial products. Reagents without specific origins indicated were commercially available conventional reagents. The structures of the compounds were determined by nuclear magnetic resonance (NMR) spectroscopy or/and mass spectrometry (MS). The NMR shifts ( 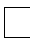 ) are given in 10-6 (ppm). The NMR analyses were performed using a Bruker AVANCE-400 nuclear magnetic resonance instrument, with dimethyl sulfoxide-D6 (DMSO-d6), chloroform-D (CDCl3), and methanol-D4 (CD3OD) as solvents, and tetramethylsilane (TMS) as an internal standard. The spatial configurations of the optical isomers (isomers) of the compounds can be further confirmed by measuring single crystal parameters.

HPLC analysis was performed on Waters ACQUITY ultra high performance LC, Shimadzu LC-20A systems, Shimadzu LC-2010HT series, or Agilent 1200 LC high performance liquid chromatograph (ACQUITY UPLC BEH C18 1.7 µm 2.1 × 50 mm column, Ultimate XB-C18 3.0 × 150 mm column, or Xtimate C18 2.1 × 30 mm column).

The MS analyses were performed on a Waters SQD2 mass spectrometer in positive/negative ion scan mode with a mass scan range of 100-1200.

The Chiral HPLC analyses were performed using a Chiralpak IC-3 100 × 4.6 mm I.D., 3 µm; Chiralpak AD-3 150 × 4.6 mm I.D., 3 µm; Chiralpak AD-3 50 × 4.6 mm I.D., 3 µm; Chiralpak AS-3 150 × 4.6 mm I.D., 3 µm; Chiralpak AS-3 100 × 4.6 mm I.D., 3 µm; ChiralCel OD-3 150 × 4.6 mm I.D., 3 µm; Chiralcel OD-3 100 × 4.6 mm I.D., 3 µm; ChiralCel OJ-H 150 × 4.6 mm I.D., 5 µm; or Chiralcel OJ-3 150 × 4.6 mm I.D., 3 µm column.

The thin-layer chromatography silica gel plates used were Yantai Huanghai HSGF254 or Qingdao GF254 silica gel plates. The silica gel plates used in the thin-layer chromatography (TLC) analyses had a layer thickness of 0.15 mm-0.2 mm, and those used in the thin-layer chromatography separations and purifications had a layer thickness of 0.4 mm-0.5 mm.

The flash column purification system used was Combiflash Rf150 (TELEDYNE ISCO) or Isolara one (Biotage).

In the normal-phase column chromatography steps, a 100-200 mesh, 200-300 mesh, or 300-400 mesh Yantai Huanghai silica gel was generally used as a carrier, or a Changzhou Santai pre-fill ultrapure normal-phase silica gel column (40-63 µm, 60 g, 12 g, 25 g, 40 g, 80 g, or other specifications) was used.

In the reversed-phase column chromatography steps, a Changzhou Santai pre-fill ultrapure C18 silica gel column (20-45 µm, 100 Å, 40 g, 80 g, 120 g, 220 g, or other specifications) was generally used. The high-pressure column purification system used was Waters AutoP equipped with a Waters XBridge BEH C18 OBD Prep Column, 130 Å, 5 µm, 19 mm × 150 mm or Atlantis T3 OBD Prep Column, 100 Å, 5 µm, 19 mm × 150 mm.

The preparative chiral columns used were DAICEL CHIRALPAK IC (250 mm × 30 mm, 10 µm) or Phenomenex-Amylose-1 (250 mm × 30 mm, 5 µm) columns.

The known starting materials in the present disclosure may be synthesized by using or following methods known in the art, or may be purchased from companies such as Shanghai Titan Scientific, ABCR GmbH & Co. KG, Acros Organics, Aldrich Chemical Company, Accela ChemBio Inc., and Chembee Chemicals.

In the examples, the reactions can all be performed under an argon atmosphere or a nitrogen atmosphere unless otherwise specified.

The argon atmosphere or nitrogen atmosphere means that the reaction flask was connected to a balloon containing about 1 L of argon or nitrogen gas.

The hydrogen atmosphere means that the reaction flask was connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 cycles of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The monitoring of the reaction progress in the examples used thin-layer chromatography (TLC). The developing solvent used in the reactions, the eluent systems used in the column chromatography purification, the developing solvent systems used in the thin-layer chromatography, and the volume ratio of the solvents were adjusted depending on the polarity of the compound, or a small amount of basic or acidic reagents such as triethylamine and acetic acid could be added for adjustment.

XRPD refers to X-ray powder diffraction: The analysis was performed using a BRUKER D8 X-ray diffractometer; specific acquisition information: a Cu anode (40 kV, 40 mA), ray: monochromatic Cu-Ka ray (l = 1.5418 Å). Scan mode: *θ*/2*θ*; scan range (2*θ* range): 5°-45°.

DSC refers to differential scanning calorimetry: The analysis was performed using a METTLER TOLEDO DSC 3+ differential scanning calorimeter, with a temperature ramp rate of 10 °C/min, specific temperature ranges shown in corresponding patterns (mostly 25-250 °C), and a nitrogen purge speed of 50 mL/min.

TGA refers to thermogravimetric analysis: The analysis was performed using a METTLER TOLEDO TGA 2 thermogravimetric analyzer, with a temperature ramp rate of 10 °C/min, specific temperature ranges shown in corresponding patterns (mostly 30-350 °C), and a nitrogen purge speed of 50 mL/min. DVS refers to dynamic vapor sorption: The analysis was performed using SMS DVS Advantage; at 25 °C, the humidity was changed as follows: 50%-95%-0%-95%-50%, in steps of 10% (5% for the last step) (specific humidity ranges are shown in corresponding patterns, and those listed here were used in most cases); the criterion was dm/dt being not greater than 0.002%.

### Example 1: Preparation of Compound of Formula 1

(*S*)-4-(3-Acetyl-5-ethoxy-2,8-dimethyl-1,4-dihydro-1,6-naphthyridin-4-yl)-3-(methoxy-*d₃*)benzonitrile **1** (with reference to the preparation method of Example 2-2 in Application No.

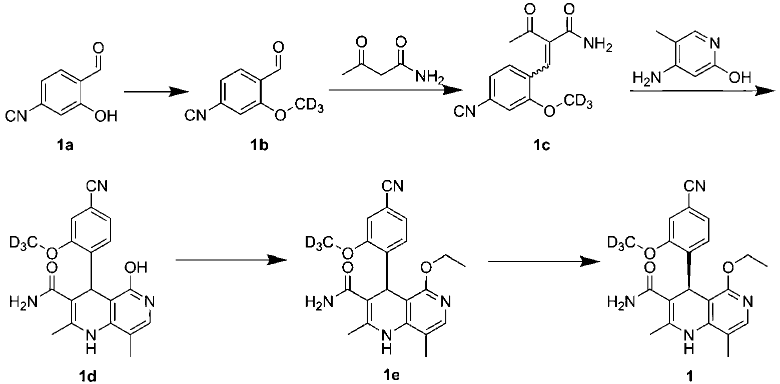

### Step 1: Synthesis of compound 1b

Compound 1a (1.0 g, 6.80 mmol) and potassium carbonate (1.0 g, 7.25 mmol) were mixed in dichloromethane (20 mL), and deuterated iodomethane (1.18 g, 8.16 mmol) was added. The mixture was stirred at room temperature until the reaction was complete. The mixture was cooled to room temperature and then diluted with water (20 mL). The mixture was extracted with ethyl acetate (20 mL × 3). The organic phases were separated, combined, washed with brine (20 mL), dried over anhydrous sodium sulfate, and then filtered. The filtrate was concentrated *in vacuo* to give a crude product, and the crude product was purified by flash column chromatography (eluent: 5-20% ethyl acetate in petroleum ether) to give compound 1b (880 mg, 78.9% yield).

1H-NMR (400 MHz, CDCl3) δ ppm 10.50 (s, 1H), 7.92 (d, J=8.0 Hz, 1H), 7.34 (d, J=8.0 Hz, 1H), 7.27 (s, 1H).

### Step 2: Synthesis of compound 1d

Compound 1b (3.0 g, 18.3 mmol) and 3-oxobutanamide (2.77 g, 27.4 mmol) were dissolved in dichloromethane (20 mL), and acetic acid (0.1 g) and morpholine (0.1 g) were added. The mixture was stirred at 40 °C until the reaction was complete. The mixture was cooled to room temperature and then filtered to give compound 1c.

Compound 1c (1.0 g, 4.1 mmol) and 4-amino-5-methylpyridin-2-ol (0.51 g, 4.1 mmol) were dissolved in isopropanol (10 mL). The mixture was stirred at 95 °C until the reaction was complete. The solvent was removed *in vacuo* to give a crude product, and the crude product was purified by silica gel flash column chromatography (eluent: 0-10% methanol in dichloromethane) to give compound 1d.

ES-MS m/z 354.2 (M+H)+.

### Step 3: Synthesis of compound 1

Compound 1d (260 mg, 0.736 mmol) was dissolved in NMP (3 mL), and sulfuric acid (36.11 mg, 0.368 mmol) and triethoxymethane (3 mL, 16.366 mmol) were added. The mixture was microwaved at 140 °C until the reaction was complete. The mixture was cooled to room temperature and then diluted with water (5 mL). The mixture was extracted with ethyl acetate (10 mL × 3). The organic phases were separated, combined, washed with brine (10 mL), dried over anhydrous sodium sulfate, and then filtered. The filtrate was concentrated *in vacuo* to give a crude product, and the crude product was purified by flash column chromatography (eluent: 0-60% ethyl acetate in petroleum ether) to give compound le (205 mg, 73.0% yield). LCMS: m/z 382.2 (M+H)⁺. ¹H NMR: (400 MHz, DMSO-d6) δ ppm 7.69 (s, 1H), 7.55 (s, 1H), 7.37 (d, J = 1.6 Hz, 1H), 7.27 (dd, J = 1.6, 8.0 Hz, 1H), 7.15 (d, J = 8.0 Hz, 1H), 6.76-6.69 (m, 2H), 5.38 (s, 1H), 4.05-3.97 (m, 2H), 2.19 (s, 3H), 2.12 (s, 3H), 1.05 (t, J = 6.8 Hz, 3H).

The product was chirally resolved (column: ChiralPak AD (150 × 4.6 mm I.D., 5 µm); mobile phases: A: supercritical CO2 fluid, B: ethanol (0.05% DEA)) to give compound 1. Retention time: 1.653 min, LCMS: 382.2 [M+H]⁺. ¹H NMR: (400 MHz, DMSO-d6) δ ppm 7.68 (s, 1H), 7.55 (s, 1H), 7.36 (d, J = 1.6 Hz, 1H), 7.27 (dd, J = 1.6, 7.6 Hz, 1H), 7.14 (d, J = 7.6 Hz, 1H), 6.86-6.55 (m, 2H), 5.37 (s, 1H), 4.08-3.95 (m, 2H), 2.18 (s, 3H), 2.12 (s, 3H), 1.04 (t, J = 7.2 Hz, 3H).

### Test Example 1: Assay for In-Vitro Antagonistic Activity Against Mineralocorticoid Receptors

### 1) Reagent preparation

All the compounds to be tested were serially diluted 3-fold from 10 mM with DMSO (Sigma, D8418), and 10 concentrations were obtained for each of the compounds.

The reference compound eplerenone was serially diluted 3-fold with DMSO to form 10 concentrations. A 1000× positive control (10 mM eplerenone) and a 1000× negative control (100% DMSO) were prepared.

### 2) Experimental procedure

2.1. All cells were cultured as recommended by ATCC. HEK293T cells were passaged and plated when in the exponential growth phase.

2.2. The old culture medium in the cell culture flask was discarded, and the cells were washed with

### PBS.

2.3. A proper amount of TrypLE solution was added to the culture flask to digest the cells to separate them, and the digestion was stopped with a serum-containing complete culture medium.

2.4. The cell suspension was centrifuged to make the cells settle. The cells were washed with PBS 2 times to remove phenol red and were then suspended with culture medium to a proper concentration (only cells with a survival rate of greater than 90% were used in the subsequent experiments).

2.5. 6 × 106 HEK293T cells were spread on a 100 mm Petri dish and incubated in a 5% CO₂ incubator at 37 °C for 16 h.

2.6. The cells were transfected with plasmids and then incubated in a 5% CO₂ incubator at 37 °C for another 5-6 h.

2.7. 25 nL of compound dilution was transferred to a 384-well assay plate using Echo655.

2.8. HEK293T cells were plated at a concentration of 17,000 cells/well in a 384-well plate, and meanwhile, 1 nM aldosterone was added to each well.

2.9. The cells were incubated in a 5% CO₂ incubator at 37 °C for 18-20 h.

2.10. 25 µL of britelite + luciferase assay reagent was added to each well of the 384-well assay plate, and luminescence values were recorded on an Envision 2105 plate reader.

2.11. The IC₅₀ values of the compounds were obtained using the software Graphad 8.0 and a non-linear fitting equation.

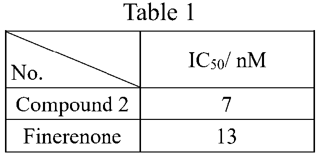

### Test Example 2: Efficacy Test in db/db Mouse Model with Diabetic Nephropathy

Proper amounts of compound **1** and finerenone were taken and separately mixed with a 0.5% hydroxyethyl cellulose solution (Tylose MH300) to form suspensions.

### 1) Grouping and administration

Thirty db/db mice (idiopathic type II diabetes model mice) were selected and divided into a model control group, a positive control group, and a treatment group, and 10 db/m mice were selected as a control group.

The mice were given the compounds by oral gavage (i.g) once daily over 4 consecutive weeks. The administration information is shown in detail in Table 2.

**Table 2. Administration information**

| Group | Route of administration | Dose of administration (mg/kg) | Concentration of administration (mg/mL) | Volume of administration (mL/kg) | Number of animals (mice) |
|---|---|---|---|---|---|
| Control group | | - | - | 10 | 10 |
| Model control group | i.g. | - | - | 10 | 10 |
| Positive control group | | 15 | 1.5 | 10 | 10 |
| Treatment group | | 15 | 1.5 | 10 | 10 |

### 2) Evaluation measures

Urine albumin was used as a main measure. The data obtained were analyzed statistically using EXCEL and IBM SPSS Statistics 22.0.

All metrology data are expressed as Mean ± SEM s. The parameters before and after administration were plotted for the different groups of animals using the software Graph Pad Prism 8. Data were analyzed using the statistical software SPSS 22.0. A Levene's test of homogeneity of variance was performed on the parameters. When the variance was homogenous (P ≥ 0.05), the Dunnett's & LSD method from the one-way analysis of variance (ANOVA) was used to compare the differences between the groups. When the variance was not homogenous (P < 0.05), the Mann-Whitney U test (the M-W method) from the Kruskal-Wallis H test (the K-W method) was used to compare the differences between the groups. For animal survival, the log-rank test from the product-limit method (the K-M method) was used to compare the differences between the groups.

### 3) Experimental results

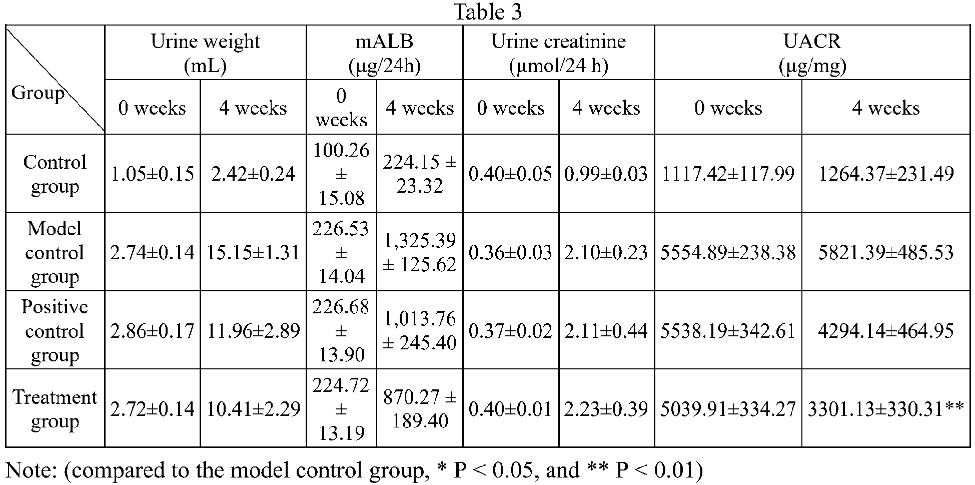

Conclusion: After 4 weeks of administration, both the urine amount and mALB in the finerenone group and the treatment group (compound 1) tended to be lower than those in the model control group. Meanwhile, the urine albumin-to-creatinine ratio (UACR) of the mice in the treatment group is statistically significantly smaller than that of the mice in the model control group (P < 0.01); the urine albumin-to-creatinine ratio (UACR) of the mice in the finerenone group decreased somewhat but is not significantly different from that of the mice in the model group, as shown in FIG. 1 (compared to the model control group, ** P < 0.01).

### Example 2: Preparation of Crystal Form I of Mesylate of Compound of Formula 1

20 mg of the compound of formula 1 was added to 0.2 mL of 90% acetone/water and completely dissolved, and 17 µL of a 3.0 M aqueous methanesulfonic acid solution was added. The mixture was crystallized, filtered, and dried *in vacuo* to give a solid. According to an X-ray powder diffraction analysis, the product was defined as the crystal form I of mesylate. Its XRPD pattern is shown in FIG. 2, and its characteristic peak positions are shown in Table 4. According to an ion chromatography analysis, the mesylate ion content of the product was 19.0%. The DSC profile shows endothermic peaks at 86.96 °C and 205.88 °C. The TGA profile shows a weight loss of 1.5% from 35 °C to 105 °C. DVS test results show that the sample had a hygroscopic weight gain of about 4.4% under normal storage conditions (i.e., 25 °C and 60% RH), a hygroscopic weight gain of about 4.9% under accelerated experimental conditions (i.e., 70% RH), and a hygroscopic weight gain of about 6.9% under extreme conditions (90% RH). After the DVS test, the crystal form was re-identified, and the results show that its crystal form did not change.

**Table 4**

| Peak No. | 2*θ* value [° or degrees] | d[Å] | Relative intensity% |
|---|---|---|---|
| 1 | 7.401 | 11.93537 | 21.3 |
| 2 | 8.635 | 10.23254 | 100.0 |
| 3 | 9.630 | 9.17706 | 9.2 |
| 4 | 14.895 | 5.94269 | 7.2 |
| 5 | 15.590 | 5.67940 | 4.2 |
| 6 | 17.457 | 5.07615 | 69.8 |
| 7 | 19.014 | 4.66364 | 7.2 |
| 8 | 19.529 | 4.54180 | 4.5 |
| 9 | 22.735 | 3.90816 | 4.3 |
| 10 | 23.662 | 3.75713 | 1.9 |
| 11 | 24.357 | 3.65145 | 2.0 |
| 12 | 26.350 | 3.37958 | 29.0 |
| 13 | 26.751 | 3.32980 | 4.9 |
| 14 | 27.833 | 3.20284 | 3.5 |
| 15 | 35.364 | 2.53613 | 2.1 |
| 16 | 38.415 | 2.34143 | 2.4 |

### Example 3: Preparation of Crystal Form I of Mesylate of Compound of Formula 1

10 mg of the compound of formula 1 was added to 0.2 mL of a solvent, and 17 µL of a 1.5 M solution of methanesulfonic acid in ethanol was added. The mixture was slurried for crystallization, filtered, and dried *in vacuo* to give a solid. According to an X-ray powder diffraction analysis, the product was the crystal form I of mesylate. The solvent is shown in Table 5 below.

**Table 5**

| Solvent | Crystal form |
|---|---|
| Isopropanol | Crystal form I of mesylate |
| Isopropyl acetate | Crystal form I of mesylate |
| 4-Methyl-2-pentanone | Crystal form I of mesylate |

### Example 4: Preparation of Crystal Form I of Mesylate of Compound of Formula 1

10 mg of the compound of formula 1 was added to 0.2 mL of acetonitrile/water (v/v = 1:1), and 17 µL of a 1.5 M solution of methanesulfonic acid in ethanol was added. The mixture was completely dissolved and volatilized for crystallization to give a solid. According to an X-ray powder diffraction analysis, the product was the crystal form I of mesylate.

### Example 5: Preparation of Crystal Form α of Maleate of Compound of Formula 1

10 mg of the compound of formula 1 and 3 mg of maleic acid were added to 1.0 mL of isopropanol. The mixture was slurried for crystallization, filtered, and dried *in vacuo* to give a solid. According to an X-ray powder diffraction analysis, the product was defined as the crystal form α of maleate. Its XRPD pattern is shown in FIG. 3, and its characteristic peak positions are shown in Table 6. According to an ion chromatography analysis, the maleate ion content of the product was 30.1%. The DSC profile shows endothermic peaks at 72.14 °C and 157.89 °C. The TGA profile shows a weight loss of 3.1% from 35 °C to 80 °C.

**Table 6**

| Peak No. | 2*θ* value [° or degrees] | d[Å] | Relative intensity% |
|---|---|---|---|
| 1 | 7.147 | 12.35835 | 100.0 |
| 2 | 8.562 | 10.31957 | 84.5 |
| 3 | 9.362 | 9.43923 | 10.3 |
| 4 | 11.015 | 8.02572 | 18.9 |
| 5 | 12.787 | 6.91754 | 19.3 |
| 6 | 14.399 | 6.14653 | 13.7 |
| 7 | 17.254 | 5.13530 | 75.0 |
| 8 | 20.869 | 4.25320 | 22.7 |
| 9 | 22.478 | 3.95227 | 29.2 |
| 10 | 26.061 | 3.41636 | 23.3 |
| 11 | 27.461 | 3.24539 | 9.2 |
| 12 | 29.638 | 3.01174 | 7.7 |
| 13 | 31.781 | 2.81335 | 1.9 |

### Example 6: Preparation of Crystal Form α of Maleate of Compound of Formula 1

10 mg of the compound of formula 1 and 3 mg of maleic acid were added to 1.0 mL of a solvent as shown in Table 7 below. The mixture was slurried for crystallization, filtered, and dried *in vacuo* to give a solid. According to an X-ray powder diffraction analysis, the product was the crystal form α of maleate.

**Table 7**

| Solvent | Crystal form |
|---|---|
| Isopropyl acetate | Crystal form α of maleate |
| 4-Methyl-2-pentanone | Crystal form α of maleate |
| 90% acetonitrile/water | Crystal form α of maleate |

### Example 7: Preparation of Crystal Form α of Maleate of Compound of Formula 1

20 mg of the compound of formula 1 was added to 0.5 mL of ethanol/dichloromethane (v/v = 1:1) and dissolved, and 66 µL of a 1.0 M aqueous maleic acid solution was added. 5 mL of n-heptane was added, and then the mixture was crystallized, filtered, and dried *in vacuo* to give a solid. According to an X-ray powder diffraction analysis, the product was the crystal form α of maleate.

### Example 8: Preparation of Crystal Form a of Sulfate of Compound of Formula 1

25 mg of the compound of formula 1 was added to 0.5 mL of 90% acetone/water and dissolved, and 36 µL of a 1.8 M aqueous sulfuric acid solution was added. The mixture was crystallized, filtered, and dried *in vacuo* to give a solid. According to an X-ray powder diffraction analysis, the product was defined as the crystal form a of sulfate. Its XRPD pattern is shown in FIG. 4, and its characteristic peak positions are shown in Table 8. According to an ion chromatography analysis, the sulfate ion content of the product was 18.6%. The DSC profile shows endothermic peaks at 109.26 °C and 182.39 °C. The TGA profile shows a weight loss of 3.9% from 35 °C to 105 °C and a weight loss of 6.2% from 160 °C to 215 °C. DVS test results show that the sample had a hygroscopic weight gain of about 1.6% under normal storage conditions (i.e., 25 °C and 60% RH), a hygroscopic weight gain of about 1.9% under accelerated experimental conditions (i.e., 70% RH), and a hygroscopic weight gain of about 3.1% under extreme conditions (90% RH). After the DVS test, the crystal form was re-identified, and the results show that its crystal form did not change.

**Table 8**

| Peak No. | 2*θ* value [° or degrees] | d[Å] | Relative intensity% |
|---|---|---|---|
| 1 | 7.130 | 12.38766 | 100.0 |
| 2 | 8.699 | 10.15706 | 71.1 |
| 3 | 9.465 | 9.33683 | 25.5 |
| 4 | 11.261 | 7.85131 | 30.5 |
| 5 | 12.333 | 7.17092 | 19.2 |
| 6 | 13.041 | 6.78315 | 8.6 |
| 7 | 14.388 | 6.15113 | 28.6 |
| 8 | 15.667 | 5.65157 | 9.8 |
| 9 | 16.826 | 5.26494 | 4.8 |
| 10 | 17.528 | 5.05556 | 92.3 |
| 11 | 18.564 | 4.77572 | 33.7 |
| 12 | 19.027 | 4.66049 | 18.2 |
| 13 | 20.842 | 4.25853 | 4.3 |
| 14 | 22.332 | 3.97781 | 50.9 |
| 15 | 22.798 | 3.89754 | 35.3 |
| 16 | 23.968 | 3.70978 | 18.1 |
| 17 | 25.091 | 3.54630 | 5.2 |
| 18 | 26.056 | 3.41704 | 16.6 |
| 19 | 26.473 | 3.36417 | 36.2 |
| 20 | 27.331 | 3.26052 | 13.3 |

### Example 9: Preparation of Crystal Form a of Sulfate of Compound of Formula 1

10 mg of the compound of formula 1 was added to 0.2 mL of a solvent, and 14 µL of a 1.8 M solution of sulfuric acid in ethanol was added. The mixture was slurried for crystallization, filtered, and dried *in vacuo* to give a solid. According to an X-ray powder diffraction analysis, the product was the crystal form a of sulfate. The solvent is shown in Table 9 below.

**Table 9**

| Solvent | Crystal form |
|---|---|
| Isopropanol | Crystal form a of sulfate |
| Isopropyl acetate | Crystal form a of sulfate |
| 4-Methyl-2-pentanone | Crystal form a of sulfate |

### Example 10: Preparation of Crystal Form a of Sulfate of Compound of Formula 1

10 mg of the compound of formula 1 was dissolved in 0.2 mL of acetonitrile/water (v/v = 1:1), and 14 µL of a 1.8 M solution of sulfuric acid in ethanol was added. The mixture was completely dissolved and volatilized for crystallization. According to an X-ray powder diffraction analysis, the product was the crystal form a of sulfate.

### Example 11: Preparation of Crystal Form a of Tartrate of Compound of Formula 1

10 mg of the compound of formula 1 and 4 mg of tartaric acid were added to 0.2 mL of isopropanol. The mixture was slurried for crystallization, filtered, and dried *in vacuo.* According to an X-ray powder diffraction analysis, the product was defined as the crystal form a of tartrate. Its XRPD pattern is shown in FIG. 5, and its characteristic peak positions are shown in Table 10. The DSC profile shows endothermic peaks at 107.47 °C, 122.48 °C, 177.96 °C, and 215.45 °C. The TGA profile shows a weight loss of 0.9% from 35 °C to 80 °C and a weight loss of 3.3% from 80 °C to 140 °C.

**Table 10**

| Peak No. | 2*θ* value [° or degrees] | d[Å] | Relative intensity% |
|---|---|---|---|
| 1 | 6.838 | 12.91730 | 23.1 |
| 2 | 7.474 | 11.81902 | 44.7 |
| 3 | 8.453 | 10.45222 | 19.6 |
| 4 | 10.546 | 8.38148 | 100.0 |
| 5 | 14.020 | 6.31173 | 21.1 |
| 6 | 15.962 | 5.54802 | 6.6 |
| 7 | 17.246 | 5.13777 | 18.4 |
| 8 | 19.022 | 4.66188 | 23.5 |
| 9 | 20.714 | 4.28470 | 41.3 |
| 10 | 21.167 | 4.19401 | 16.5 |
| 11 | 21.677 | 4.09641 | 16.4 |
| 12 | 22.866 | 3.88597 | 27.4 |
| 13 | 23.888 | 3.72198 | 12.3 |
| 14 | 25.520 | 3.48759 | 24.1 |
| 15 | 26.461 | 3.36569 | 17.5 |
| 16 | 27.173 | 3.27913 | 20.5 |
| 17 | 27.835 | 3.20260 | 12.3 |
| 18 | 28.243 | 3.15723 | 2.1 |
| 19 | 28.821 | 3.09518 | 7.1 |
| 20 | 29.570 | 3.01852 | 1.8 |

### Example 12: Preparation of Crystal Form b of Tartrate of Compound of Formula 1

101 mg of the compound of formula 1 and 42 mg of tartaric acid were added to 1 mL of 90% acetonitrile/water. The mixture was slurried for crystallization, filtered, and dried *in vacuo* to give a solid. According to an X-ray powder diffraction analysis, the product was defined as the crystal form b of tartrate. Its XRPD pattern is shown in FIG. 6, and its characteristic peak positions are shown in Table 11. According to an ion chromatography analysis, the tartrate ion content of the product was 29.0%. The DSC profile shows an endothermic peak at 179.16 °C. The TGA profile shows a weight loss of 0.1% from 35 °C to 115 °C. DVS test results show that the sample had a hygroscopic weight gain of about 4.1% under normal storage conditions (i.e., 25 °C and 60% RH), a hygroscopic weight gain of about 4.3% under accelerated experimental conditions (i.e., 70% RH), and a hygroscopic weight gain of about 5.6% under extreme conditions (90% RH). After the DVS test, the crystal form was re-identified, and the results show that its crystal form did not change.

**Table 11**

| Peak No. | 2*θ* value [° or degrees] | d[Å] | Relative intensity% |
|---|---|---|---|
| 1 | 6.823 | 12.94506 | 20.4 |
| 2 | 7.456 | 11.84759 | 18.3 |
| 3 | 8.674 | 10.18657 | 85.8 |
| 4 | 9.822 | 8.99763 | 30.8 |
| 5 | 11.217 | 7.88193 | 15.0 |
| 6 | 11.826 | 7.47720 | 35.9 |
| 7 | 12.321 | 7.17774 | 19.4 |
| 8 | 13.041 | 6.78315 | 8.4 |
| 9 | 15.783 | 5.61035 | 5.7 |
| 10 | 17.482 | 5.06878 | 100.0 |
| 11 | 19.798 | 4.48081 | 20.3 |
| 12 | 20.534 | 4.32191 | 8.8 |
| 13 | 21.962 | 4.04383 | 10.6 |
| 14 | 22.566 | 3.93710 | 51.4 |
| 15 | 23.584 | 3.76926 | 14.5 |
| 16 | 25.602 | 3.47666 | 13.9 |
| 17 | 26.431 | 3.36938 | 28.8 |
| 18 | 27.215 | 3.27414 | 12.0 |
| 19 | 27.755 | 3.21158 | 13.6 |
| 20 | 29.957 | 2.98040 | 3.9 |

### Example 13: Preparation of Crystal Form b of Tartrate of Compound of Formula 1

20 mg of the compound of formula 1 was added to 0.5 mL of ethanol/dichloromethane (v/v = 1:1) and dissolved, and 66 µL of a 1.0 M aqueous tartaric acid solution was added. 5 mL of n-heptane was added, and then the mixture was crystallized, filtered, and dried *in vacuo.* According to an X-ray powder diffraction analysis, the product was the crystal form b of tartrate.

### Example 14: Preparation of Crystal Form I of Hydrochloride of Compound of Formula 1

60 mg of the compound of formula 1 was added to 1.0 mL of isopropanol, and 137 µL of a 1.2 M solution of hydrochloric acid in ethanol was added. The mixture was slurried for crystallization, filtered, and dried *in vacuo* to give a solid. According to an X-ray powder diffraction analysis, the product was defined as the crystal form I of hydrochloride. Its XRPD pattern is shown in FIG. 7, and its characteristic peak positions are shown in Table 12. According to an ion chromatography analysis, the chloride ion content of the product was 9.1%. The DSC profile shows an endothermic peak at 208.60 °C. The TGA profile shows a weight loss of 0.1% from 35 °C to 150 °C and a weight loss of 10.2% from 180 °C to 220 °C. DVS test results show that the sample had a hygroscopic weight gain of about 3.6% under normal storage conditions (i.e., 25 °C and 60% RH), a hygroscopic weight gain of about 13.7% under accelerated experimental conditions (i.e., 70% RH), and a hygroscopic weight gain of about 37.5% under extreme conditions (90% RH). After the DVS test, the crystal form was re-identified, and the results show that its crystal form did not change.

**Table 12**

| Peak No. | 2*θ* value [° or degrees] | d[Å] | Relative intensity% |
|---|---|---|---|
| 1 | 5.266 | 16.76826 | 19.2 |
| 2 | 7.585 | 11.64555 | 86.7 |
| 3 | 9.893 | 8.93345 | 27.2 |
| 4 | 10.560 | 8.37103 | 100.0 |
| 5 | 11.394 | 7.75957 | 22.8 |
| 6 | 14.873 | 5.95169 | 21.6 |
| 7 | 15.645 | 5.65949 | 31.0 |
| 8 | 17.058 | 5.19393 | 13.1 |
| 9 | 17.447 | 5.07905 | 23.1 |
| 10 | 19.874 | 4.46383 | 10.9 |
| 11 | 20.379 | 4.35432 | 4.4 |
| 12 | 20.997 | 4.22755 | 7.1 |
| 13 | 21.345 | 4.15948 | 5.5 |
| 14 | 21.769 | 4.07926 | 9.8 |
| 15 | 22.452 | 3.95677 | 55.3 |
| 16 | 23.008 | 3.86231 | 30.6 |
| 17 | 24.627 | 3.61197 | 6.6 |
| 18 | 26.404 | 3.37284 | 9.1 |
| 19 | 26.790 | 3.32509 | 5.2 |
| 20 | 27.215 | 3.27414 | 7.4 |
| 21 | 27.841 | 3.20191 | 19.2 |
| 22 | 29.686 | 3.00692 | 6.0 |
| 23 | 30.150 | 2.96174 | 11.7 |
| 24 | 30.691 | 2.91079 | 2.8 |
| 25 | 31.193 | 2.86508 | 6.7 |
| 26 | 31.695 | 2.82083 | 3.8 |
| 27 | 32.313 | 2.76828 | 5.3 |
| 28 | 33.162 | 2.69927 | 3.2 |
| 29 | 35.209 | 2.54690 | 4.5 |

### Example 15: Preparation of Crystal Form I of Hydrochloride of Compound of Formula 1

60 mg of the compound of formula 1 was added to 1.0 mL of a solvent as shown in Table 13 below. 137 µL of a 1.2 M solution of hydrochloric acid in ethanol was added. The mixture was slurried for crystallization, filtered, and dried *in vacuo* to give a solid. According to an X-ray powder diffraction analysis, the product was the crystal form I of hydrochloride.

**Table 13**

| Solvent | Crystal form |
|---|---|
| Isopropyl acetate | Crystal form I of hydrochloride |
| 4-Methyl-2-pentanone | Crystal form I of hydrochloride |

### Example 16: Preparation of Crystal Form I of Hydrochloride of Compound of Formula 1

10 mg of the compound of formula 1 was added to 0.2 mL of acetonitrile/water (v/v = 1:1), and 22 µL of a 1.2 M solution of hydrochloric acid in ethanol was added. The mixture was completely dissolved and volatilized for crystallization. According to an X-ray powder diffraction analysis, the product was the crystal form I of hydrochloride.

### Example 17: Preparation of Crystal Form a of p-Toluenesulfonate of Compound of Formula 1

20 mg of the compound of formula 1 and 9 mg of p-toluenesulfonic acid were added to 1.0 mL of isopropanol. The mixture was slurried for crystallization, centrifuged, and dried *in vacuo* to give a solid. According to an X-ray powder diffraction analysis, the product was defined as the crystal form a of p-toluenesulfonate. Its XRPD pattern is shown in FIG. 8, and its characteristic peak positions are shown in Table 14. According to an ion chromatography analysis, the p-toluenesulfonate ion content of the product was 31.8%. The DSC profile shows an endothermic peak at 164.37 °C. The TGA profile shows a weight loss of 4.1% from 35 °C to 140 °C.

**Table 14**

| Peak No. | 2*θ* value [° or degrees] | d[Å] | Relative intensity% |
|---|---|---|---|
| 1 | 5.163 | 17.10107 | 100.0 |
| 2 | 6.628 | 13.32469 | 15.6 |
| 3 | 7.967 | 11.08841 | 2.9 |
| 4 | 8.954 | 9.86868 | 2.4 |
| 5 | 10.421 | 8.48182 | 54.0 |
| 6 | 10.934 | 8.08549 | 19.4 |
| 7 | 13.342 | 6.63083 | 2.3 |
| 8 | 15.009 | 5.89791 | 2.4 |
| 9 | 16.744 | 5.29047 | 4.3 |
| 10 | 21.847 | 4.06490 | 1.8 |
| 11 | 23.072 | 3.85182 | 3.2 |
| 12 | 24.762 | 3.59259 | 7.7 |

### Example 18: Preparation of Crystal Form b of p-Toluenesulfonate of Compound of Formula 1

20 mg of the compound of formula 1 and 10 mg of p-toluenesulfonic acid were added to 1.0 mL of 90% acetonitrile/water. The mixture was slurried for crystallization, filtered, and dried *in vacuo* to give a solid. According to an X-ray powder diffraction analysis, the product was defined as the crystal form b of p-toluenesulfonate. Its XRPD pattern is shown in FIG. 9, and its characteristic peak positions are shown in Table 15. According to an ion chromatography analysis, the p-toluenesulfonate ion content of the product was 30.8%. The DSC profile shows an endothermic peak at 169.80 °C. The TGA profile shows a weight loss of 5.6% from 35 °C to 105 °C.

**Table 15**

| Peak No. | 2*θ* value [° or degrees] | d[Å] | Relative intensity% |
|---|---|---|---|
| 1 | 8.410 | 10.50478 | 100.0 |
| 2 | 9.493 | 9.30928 | 10.8 |
| 3 | 10.794 | 8.19003 | 2.3 |
| 4 | 11.535 | 7.66514 | 3.2 |
| 5 | 13.727 | 6.44562 | 6.0 |
| 6 | 15.299 | 5.78680 | 1.8 |
| 7 | 16.937 | 5.23053 | 1.8 |
| 8 | 17.760 | 4.98996 | 3.4 |
| 9 | 18.343 | 4.83275 | 1.8 |
| 10 | 18.751 | 4.72845 | 1.2 |
| 11 | 19.199 | 4.61930 | 2.2 |
| 12 | 22.196 | 4.00182 | 6.8 |
| 13 | 23.296 | 3.81528 | 3.4 |
| 14 | 23.964 | 3.71039 | 3.2 |
| 15 | 24.857 | 3.57903 | 7.8 |
| 16 | 25.144 | 3.53888 | 8.1 |
| 17 | 26.312 | 3.38434 | 6.4 |
| 18 | 26.882 | 3.31389 | 1.6 |
| 19 | 28.234 | 3.15824 | 3.0 |
| 20 | 28.991 | 3.07741 | 1.7 |
| 21 | 33.788 | 2.65068 | 1.4 |

### Example 19: Preparation of Crystal Form a of Citrate of Compound of Formula 1

10 mg of the compound of formula 1 and 6 mg of citric acid were added to 0.2 mL of 90% acetonitrile/water. The mixture was volatilized for crystallization. According to an X-ray powder diffraction analysis, the product was defined as the crystal form a of citrate. Its XRPD pattern is shown in FIG. 10, and its characteristic peak positions are shown in Table 16. The DSC profile shows endothermic peaks at 147.44 °C and 169.42 °C. The TGA profile shows a weight loss of 2.8% from 35 °C to 120 °C.

**Table 16**

| Peak No. | 2*θ* value [° or degrees] | d[Å] | Relative intensity% |
|---|---|---|---|
| 1 | 7.218 | 12.23651 | 93.1 |
| 2 | 7.899 | 11.18323 | 43.5 |
| 3 | 9.479 | 9.32229 | 42.6 |
| 4 | 11.307 | 7.81935 | 35.6 |
| 5 | 11.757 | 7.52126 | 100.0 |
| 6 | 15.111 | 5.85831 | 24.1 |
| 7 | 15.875 | 5.57800 | 26.7 |
| 8 | 17.927 | 4.94409 | 34.2 |
| 9 | 18.887 | 4.69469 | 9.0 |
| 10 | 19.942 | 4.44873 | 12.1 |
| 11 | 21.133 | 4.20068 | 13.7 |
| 12 | 22.498 | 3.94873 | 39.0 |
| 13 | 23.888 | 3.72198 | 6.4 |
| 14 | 25.453 | 3.49658 | 6.6 |
| 15 | 27.461 | 3.24539 | 14.6 |
| 16 | 28.175 | 3.16470 | 7.9 |
| 17 | 29.400 | 3.03559 | 5.0 |

### Example 20: Preparation of Crystal Form I of Malate of Compound of Formula 1

60 mg of the compound of formula 1 and 22 mg of malic acid were added to 1.0 mL of isopropyl acetate. The mixture was slurried for crystallization, filtered, and dried *in vacuo.* According to an X-ray powder diffraction analysis, the product was defined as the crystal form I of malate. Its XRPD pattern is shown in FIG. 11, and its characteristic peak positions are shown in Table 17. According to an ion chromatography analysis, the malate ion content of the product was 25.1%. The DSC profile shows endothermic peaks at 90.39 °C and 184.24 °C. The TGA profile shows a weight loss of 4.0% from 35 °C to 115 °C.

**Table 17**

| Peak No. | 2*θ* value [° or degrees] | d[Å] | Relative intensity% |
|---|---|---|---|
| 1 | 7.194 | 12.27780 | 72.4 |
| 2 | 8.307 | 10.63506 | 11.3 |
| 3 | 10.779 | 8.20131 | 29.9 |
| 4 | 11.339 | 7.79711 | 21.3 |
| 5 | 12.996 | 6.80642 | 13.5 |
| 6 | 14.499 | 6.10433 | 3.6 |
| 7 | 15.263 | 5.80050 | 19.4 |
| 8 | 16.574 | 5.34439 | 4.1 |
| 9 | 17.357 | 5.10517 | 5.4 |
| 10 | 19.806 | 4.47898 | 0.4 |
| 11 | 20.963 | 4.23438 | 7.2 |
| 12 | 21.782 | 4.07695 | 100.0 |
| 13 | 26.882 | 3.31389 | 2.8 |
| 14 | 27.767 | 3.21030 | 2.9 |
| 15 | 29.155 | 3.06055 | 10.2 |

### Example 21: Preparation of Crystal Form I of Malate of Compound of Formula 1

10 mg of the compound of formula 1 and 3 mg of malic acid were added to 0.2 mL of 4-methyl-2-pentanone. The mixture was slurried for crystallization, filtered, and dried *in vacuo.* According to an X-ray powder diffraction analysis, the product was the crystal form I of malate.

### Example 22: Preparation of Crystal Form II of Malate of Compound of Formula 1

10 mg of the compound of formula 1 and 3 mg of malic acid were added to 0.2 mL of 90% acetonitrile/water. The mixture was completely dissolved and volatilized for crystallization to give a solid. According to an X-ray powder diffraction analysis, the product was defined as the crystal form II of malate. Its XRPD pattern is shown in FIG. 12, and its characteristic peak positions are shown in Table 18. The DSC profile shows endothermic peaks at 71.80 °C and 138.26 °C. The TGA profile shows a weight loss of 2.8% from 30 °C to 120 °C.

**Table 18**

| Peak No. | 2*θ* value [° or degrees] | d[Å] | Relative intensity% |
|---|---|---|---|
| 1 | 4.264 | 20.70522 | 21.2 |
| 2 | 7.177 | 12.30654 | 100.0 |
| 3 | 8.583 | 10.29443 | 31.5 |
| 4 | 9.389 | 9.41233 | 6.0 |
| 5 | 11.173 | 7.91311 | 9.0 |
| 6 | 11.548 | 7.65661 | 11.4 |
| 7 | 12.752 | 6.93632 | 24.2 |
| 8 | 14.419 | 6.13793 | 8.1 |
| 9 | 16.590 | 5.33916 | 10.0 |
| 10 | 17.248 | 5.13694 | 37.8 |
| 11 | 18.023 | 4.91782 | 18.3 |
| 12 | 19.036 | 4.65849 | 6.3 |
| 13 | 19.696 | 4.50382 | 12.8 |
| 14 | 20.863 | 4.25437 | 14.1 |
| 15 | 21.629 | 4.10533 | 3.7 |
| 16 | 22.530 | 3.94319 | 40.0 |
| 17 | 23.333 | 3.80936 | 15.8 |
| 18 | 24.766 | 3.59207 | 8.6 |
| 19 | 25.623 | 3.47376 | 3.1 |
| 20 | 26.100 | 3.41137 | 8.1 |
| 21 | 27.328 | 3.26078 | 13.1 |
| 22 | 27.757 | 3.21145 | 10.4 |
| 23 | 28.753 | 3.10235 | 2.4 |
| 24 | 29.196 | 3.05635 | 3.3 |
| 25 | 29.614 | 3.01414 | 6.4 |
| 26 | 30.761 | 2.90433 | 2.6 |
| 27 | 31.846 | 2.80776 | 6.6 |
| 28 | 33.788 | 2.65068 | 2.2 |
| 29 | 35.025 | 2.55986 | 5.7 |

### Example 23: Preparation of Crystal Form III of Malate of Compound of Formula 1

The crystal form II of malate of the compound of formula 1 was heated to 110 °C. According to an X-ray powder diffraction analysis, the product was defined as the crystal form III of malate. Its XRPD pattern is shown in FIG. 13, and its characteristic peak positions are shown in Table 19. The DSC profile shows endothermic peaks at 64.13 °C and 138.90 °C. The TGA profile shows a weight loss of 1.3% from 30 °C to 100 °C.

**Table 19**

| Peak No. | 2*θ* value [° or degrees] | d[Å] | Relative intensity% |
|---|---|---|---|
| 1 | 6.847 | 12.89859 | 100.0 % |
| 2 | 8.609 | 10.26300 | 38.5 % |
| 3 | 9.815 | 9.00459 | 17.9 % |
| 4 | 11.179 | 7.90832 | 28.0 % |
| 5 | 17.410 | 5.08962 | 33.2 % |
| 6 | 19.023 | 4.66164 | 3.5 % |
| 7 | 20.927 | 4.24145 | 9.6 % |
| 8 | 22.494 | 3.94954 | 50.5 % |
| 9 | 27.351 | 3.25810 | 9.8 % |

### Example 24: Preparation of Crystal Form α of Phosphate of Compound of Formula 1

10 mg of the compound of formula 1 was added to 0.2 mL of 4-methyl-2-pentanone, and 18 µL of a 1.5 M solution of phosphoric acid in ethanol was added. The mixture was slurried for crystallization, filtered, and dried *in vacuo* to give a solid. According to an X-ray powder diffraction analysis, the product was defined as the crystal form α of phosphate. Its XRPD pattern is shown in FIG. 14, and its characteristic peak positions are shown in Table 20. According to an ion chromatography analysis, the phosphate ion content of the product was 25.0%. The DSC profile shows endothermic peaks at 57.66 °C and 144.64 °C. The TGA profile shows a weight loss of 2.3% from 35 °C to 95 °C and a weight loss of 1.4% from 95 °C to 150 °C.

**Table 20**

| Peak No. | 2*θ* value [° or degrees] | d[Å] | Relative intensity% |
|---|---|---|---|
| 1 | 6.785 | 13.01759 | 44.8 |
| 2 | 8.021 | 11.01436 | 33.2 |
| 3 | 9.756 | 9.05834 | 60.3 |
| 4 | 11.072 | 7.98505 | 59.6 |
| 5 | 12.771 | 6.92613 | 28.9 |
| 6 | 17.455 | 5.07662 | 66.5 |
| 7 | 20.688 | 4.28998 | 21.4 |
| 8 | 22.489 | 3.95037 | 100.0 |
| 9 | 23.430 | 3.79376 | 14.5 |
| 10 | 24.936 | 3.56792 | 17.9 |
| 11 | 25.670 | 3.46757 | 3.8 |
| 12 | 27.369 | 3.25601 | 11.2 |

### Example 25: Preparation of Crystal Form α of Phosphate of Compound of Formula 1

10 mg of the compound of formula 1 was added to 0.2 mL of isopropyl acetate, and 18 µL of a 1.5 M solution of phosphoric acid in ethanol was added. The mixture was slurried for crystallization, filtered, and dried *in vacuo* to give a solid. According to an X-ray powder diffraction analysis, the product was the crystal form α of phosphate.

### Example 26: Preparation of Crystal Form α of Phosphate of Compound of Formula 1

10 mg of the compound of formula 1 was added to 0.2 mL of acetonitrile/water (v/v = 1:1), and 18 µL of a 1.5 M solution of phosphoric acid in ethanol was added. The mixture was completely dissolved and volatilized for crystallization. According to an X-ray powder diffraction analysis, the product was the crystal form α of phosphate.

### Example 27: Preparation of Crystal Form I of Hydrobromide of Compound of Formula 1

10 mg of the compound of formula 1 was added to 0.2 mL of isopropanol, and 35 µL of a 0.8 M solution of hydrobromic acid in ethanol was added. The mixture was slurried for crystallization, filtered, and dried *in vacuo* to give a solid. According to an X-ray powder diffraction analysis, the product was defined as the crystal form I of hydrobromide. Its XRPD pattern is shown in FIG. 15, and its characteristic peak positions are shown in Table 21. According to an ion chromatography analysis, the hydrobromide ion content of the product was 17.6%. The DSC profile shows an endothermic peak at 207.68 °C. The TGA profile shows a weight loss of 0.3% from 35 °C to 105 °C. DVS test results show that the sample had a hygroscopic weight gain of about 12.8% under normal storage conditions (i.e., 25 °C and 60% RH), a hygroscopic weight gain of about 13.2% under accelerated experimental conditions (i.e., 70% RH), and a hygroscopic weight gain of about 13.8% under extreme conditions (90% RH). After the DVS test, the crystal form was re-identified, and the results show that its crystal form did not change.

**Table 21**

| Peak No. | 2*θ* value [° or degrees] | d[Å] | Relative intensity% |
|---|---|---|---|
| 1 | 5.089 | 17.35193 | 63.3 |
| 2 | 7.489 | 11.79463 | 88.8 |
| 3 | 10.392 | 8.50582 | 100.0 |
| 4 | 11.377 | 7.77118 | 18.4 |
| 5 | 13.361 | 6.62147 | 7.6 |
| 6 | 14.615 | 6.05587 | 23.6 |
| 7 | 15.112 | 5.85786 | 20.9 |
| 8 | 17.344 | 5.10881 | 8.9 |
| 9 | 19.581 | 4.53001 | 24.4 |
| 10 | 20.566 | 4.31508 | 15.7 |
| 11 | 21.289 | 4.17025 | 7.7 |
| 12 | 22.377 | 3.96983 | 75.5 |
| 13 | 22.936 | 3.87436 | 28.5 |
| 14 | 24.337 | 3.65434 | 10.1 |
| 15 | 25.013 | 3.55718 | 7.5 |
| 16 | 26.581 | 3.35077 | 14.7 |
| 17 | 27.154 | 3.28129 | 10.4 |
| 18 | 27.675 | 3.22075 | 20.1 |
| 19 | 28.650 | 3.11333 | 7.3 |
| 20 | 29.592 | 3.01627 | 30.5 |
| 21 | 31.726 | 2.81812 | 11.3 |
| 22 | 32.972 | 2.71443 | 6.4 |
| 23 | 35.455 | 2.52978 | 4.4 |
| 24 | 36.102 | 2.48595 | 2.9 |
| 25 | 37.190 | 2.41564 | 1.8 |
| 26 | 39.300 | 2.29071 | 3.7 |

### Example 28: Preparation of Crystal Form I of Hydrobromide of Compound of Formula 1

10 mg of the compound of formula 1 was added to 0.2 mL of a solvent as shown in Table 22. 35 µL of a 0.8 M solution of hydrobromic acid in ethanol was added. The mixture was slurried for crystallization, filtered, and dried *in vacuo* to give a solid. According to an X-ray powder diffraction analysis, the product was the crystal form I of hydrobromide.

**Table 22**

| Solvent | Crystal form |
|---|---|
| Isopropyl acetate | Crystal form I of hydrobromide |
| 4-Methyl-2-pentanone | Crystal form I of hydrobromide |

### Example 29: Preparation of Crystal Form I of Hydrobromide of Compound of Formula 1

10 mg of the compound of formula 1 was added to 0.2 mL of 90% acetonitrile/water, and 35 µL of a 0.8 M solution of hydrobromic acid in ethanol was added. The mixture was completely dissolved and volatilized for crystallization. According to an X-ray powder diffraction analysis, the product was the crystal form I of hydrobromide.

### Example 30: Preparation of Crystal Form I of Hydrobromide of Compound of Formula 1

25 mg of the compound of formula 1 was added to 0.5 mL of methanol/dichloromethane (v/v = 1:1), and 88 µL of a 0.8 M solution of hydrobromic acid in ethanol was added. The mixture was completely dissolved, and 1 mL of ethyl acetate was added. The mixture was slurried for crystallization. According to an X-ray powder diffraction analysis, the product was the crystal form I of hydrobromide.

### Example 31: Preparation of Crystal Form A of Compound of Formula 1

5 mg of the compound of formula 1 was added to 1.0 mL of water. The mixture was slurried for crystallization, filtered, and dried *in vacuo* to give a solid. According to an X-ray powder diffraction analysis, the product was defined as the crystal form A. Its XRPD pattern is shown in FIG. 16, and its characteristic peak positions are shown in Table 23. The DSC profile shows an endothermic peak at 249.27 °C. The TGA profile shows a weight loss of 1.0% from 35 °C to 160 °C.

DVS test results show that the sample had a hygroscopic weight gain of about 0.5% under normal storage conditions (i.e., 25 °C and 60% RH), a hygroscopic weight gain of about 0.7% under accelerated experimental conditions (i.e., 70% RH), and a hygroscopic weight gain of about 1.5% under extreme conditions (90% RH). In the process of the humidity changing from 0% to 95% RH, the desorption process of the sample overlapped with its adsorption process. After the DVS test, the crystal form was re-identified, and the results show that its crystal form did not change.

**Table 23**

| Peak No. | 2*θ* value [° or degrees] | d[Å] | Relative intensity% |
|---|---|---|---|
| 1 | 8.459 | 10.44489 | 74.1 |
| 2 | 11.265 | 7.84860 | 13.5 |
| 3 | 13.248 | 6.67756 | 19.7 |
| 4 | 13.964 | 6.33694 | 100.0 |
| 5 | 15.319 | 5.77932 | 21.6 |
| 6 | 15.860 | 5.58320 | 11.1 |
| 7 | 17.119 | 5.17562 | 39.6 |
| 8 | 18.987 | 4.67037 | 97.4 |
| 9 | 19.709 | 4.50085 | 20.7 |
| 10 | 20.481 | 4.33287 | 27.0 |
| 11 | 21.962 | 4.04383 | 3.3 |
| 12 | 22.914 | 3.87801 | 40.2 |
| 13 | 23.874 | 3.72421 | 19.0 |
| 14 | 25.530 | 3.48623 | 51.0 |
| 15 | 26.428 | 3.36975 | 42.6 |
| 16 | 27.292 | 3.26505 | 7.5 |
| 17 | 28.184 | 3.16369 | 16.8 |
| 18 | 28.721 | 3.10577 | 8.2 |
| 19 | 29.571 | 3.01844 | 6.8 |
| 20 | 30.497 | 2.92878 | 8.0 |

### Example 32: Preparation of Crystal Form A of Compound of Formula 1

5 mg of the compound of formula 1 was added to 1.0 mL of a solvent as shown in Table 24 below. The mixture was slurried for crystallization, filtered, and dried *in vacuo* to give a solid. According to an X-ray powder diffraction analysis, the product was the crystal form A.

**Table 24**

| Solvent | Crystal form |
|---|---|
| Isopropanol | A |
| Isopropyl acetate | A |
| Methyl tert-butyl ether | A |
| Isopropyl ether | A |
| Toluene | A |
| n-Heptane | A |
| Ethyl acetate | A |
| 4-Methyl-2-pentanone | A |
| Acetonitrile | A |

### Example 33: Preparation of Crystal Form A of Compound of Formula 1

5 mg of the compound of formula 1 was added to 0.1 mL of a solvent A and dissolved, and 1.0 mL of a solvent B was added, wherein the solvent A and the solvent B are shown in Table 25 below. The mixture was crystallized, filtered, and dried *in vacuo* to give a solid. According to an X-ray powder diffraction analysis, the product was the crystal form A.

**Table 25**

| Solvent A | Solvent B | Crystal form |
|---|---|---|
| Dimethyl sulfoxide | Water | A |
| Methanol | Isopropyl ether | A |
| Dichloromethane | n-Heptane | A |
| Acetonitrile | Methyl tert-butyl ether | A |
| Dimethyl sulfoxide | Isopropyl acetate | A |

### Example 34: Preparation of Amorphous Form of Compound of Formula 1

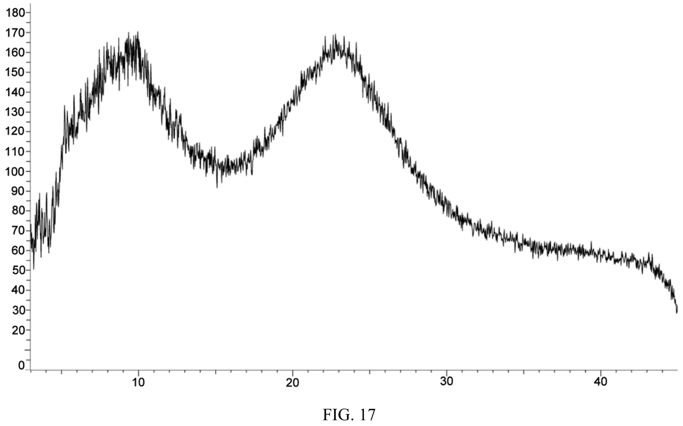

5 mg of the compound of formula 1 was added to 1.0 mL of a solvent, and the mixture was volatilized for crystallization. The solvent is shown in Table 26 below. According to an X-ray powder diffraction analysis, the product was an amorphous form. Its XRPD pattern is shown in FIG. 17.

**Table 26**

| Solvent | Crystal form |
|---|---|
| 2-Butanone | Amorphous form |
| Methyl isobutyl ketone | Amorphous form |
| Acetonitrile | Amorphous form |

### Example 35: Preparation of Amorphous Form of Compound of Formula 1

5 mg of the compound of formula 1 was added to 0.3 mL of a solvent, and the mixture was volatilized for crystallization. The solvent is shown in Table 27 below. According to an X-ray powder diffraction analysis, the product was an amorphous form.

**Table 27**

| Solvent | Crystal form |
|---|---|
| Dichloromethane | Amorphous form |
| Chloroform | Amorphous form |
| Ethanol | Amorphous form |
| Tetrahydrofuran | Amorphous form |
| Acetone | Amorphous form |

### Example 36: Stability Study of Crystal Form A

Samples of the crystal form A were taken, spread in open containers, and left to stand under conditions of light exposure (4500 Lux), high temperatures (40 °C and 60 °C), and high humidities (75% RH and 92.5% RH) for 30 days to study its stability.

**Table 28. Influencing factors of crystal form A**

| **Condition** | **Time** | **Purity (%)** | **Crystal form** |
|---|---|---|---|
| / | 0 days | 99.7 | Crystal form A |
| 40 °C | 7 days | 99.6 | No change |
| | 14 days | 99.6 | No change |
| | 1 month | 99.4 | No change |
| 60 °C | 7 days | 99.7 | No change |
| | 14 days | 99.6 | No change |
| | 1 month | 99.4 | No change |
| 75% RH | 7 days | 99.6 | No change |
| | 14 days | 99.7 | No change |
| | 1 month | 99.7 | No change |
| 92.5% RH | 7 days | 99.6 | No change |
| | 14 days | 99.7 | No change |
| | 1 month | 99.7 | No change |
| Light exposure | 7 days | 99.4 | No change |
| | 14 days | 99.1 | No change |
| | 1 month | 97.6 | No change |

The influencing factor experiment shows that the crystal form A was degraded under the light exposure condition, and was stable under other conditions.

### Example 37: Long-Term/Accelerated Stability Study of Crystal Form A

Samples of the crystal form A were sealed in aluminum foil bags and left to stand under conditions of 25 °C/60% RH and 40 °C/75% RH to study its stability.

**Table 29. Long-term accelerated stability of crystal form A**

| **Condition** | **Time** | **Purity (%)** | **Crystal form** |
|---|---|---|---|
| / | 0 days | 99.7 | Crystal form A |
| 25 °C-60% RH | 1 month | 99.7 | No change |
| | 2 months | 99.7 | No change |
| | 3 months | 99.7 | No change |
| | 6 months | 99.7 | No change |
| 40 °C-75% RH | 1 month | 99.7 | No change |
| | 2 months | 99.6 | No change |
| | 3 months | 99.6 | No change |
| | 6 months | 99.5 | No change |

Conclusion: The long-term/accelerated stability study shows that the crystal form A exhibited good physical and chemical stability after storage under long-term accelerated conditions for 6 months.

### Example 38: Stability Study of Salt Forms

Samples of the crystal form I of mesylate, crystal form b of tartrate, crystal form a of sulfate, crystal form α of maleate, crystal form I of hydrobromide, and crystal form I of hydrochloride were taken, spread in open containers, and left to stand under conditions of light exposure (4500 Lux), high temperatures (40 °C and 60 °C), and high humidities (75% RH and 92.5% RH) for 30 days to study their stability.

**Table 30. Influencing factors of crystal form I of mesylate**

| **Condition** | **Time** | **Purity (%)** | **Crystal form** |
|---|---|---|---|
| / | 0 days | 99.8 | Crystal form I of mesylate |
| 40°C | 7 days | 99.8 | No change |
| | 14 days | 99.7 | No change |
| | 1 month | 99.7 | No change |
| 60°C | 7 days | 99.8 | No change |
| | 14 days | 99.7 | No change |
| | 1 month | 99.6 | No change |
| 75% RH | 7 days | 99.8 | No change |
| | 14 days | 99.8 | No change |
| | 1 month | 99.8 | No change |
| 92.5% RH | 7 days | 99.8 | No change |
| | 14 days | 99.7 | No change |
| | 1 month | 99.8 | No change |
| Light exposure | 7 days | 99.7 | No change |
| | 14 days | 99.6 | No change |
| | 1 month | 99.5 | No change |

**Table 31. Influencing factors of crystal form b of tartrate**

| **Condition** | **Time** | **Purity (%)** | **Crystal form** |
|---|---|---|---|
| / | 0 days | 99.8 | Crystal form b of tartrate |
| 40°C | 7 days | 97.9 | No change |
| | 14 days | 97.3 | No change |
| | 1 month | 96.9 | No change |
| 60°C | 7 days | 98.2 | No change |
| | 14 days | 97.1 | No change |
| | 1 month | 96.0 | No change |
| 75% RH | 7 days | 99.7 | No change |
| | 14 days | 99.6 | No change |
| | 1 month | 99.6 | No change |
| 92.5% RH | 7 days | 99.7 | No change |
| | 14 days | 99.7 | No change |
| | 1 month | 99.7 | No change |
| Light exposure | 7 days | 98.8 | No change |
| | 14 days | 98.1 | No change |
| | 1 month | 97.0 | No change |

**Table 32. Influencing factors of crystal form a of sulfate**

| **Condition** | **Time** | **Purity (%)** | **Crystal form** |
|---|---|---|---|
| / | 0 days | 99.7 | Crystal form a of sulfate |
| 40°C | 7 days | 99.4 | No change |
| | 14 days | 98.8 | No change |
| | 1 month | 98.5 | No change |
| 60°C | 7 days | 99.2 | No change |
| | 14 days | 98.7 | No change |
| | 1 month | 97.9 | No change |
| 75% RH | 7 days | 99.7 | No change |
| | 14 days | 99.6 | No change |
| | 1 month | 99.7 | No change |
| 92.5% RH | 7 days | 99.6 | No change |
| | 14 days | 99.7 | No change |
| | 1 month | 99.7 | No change |
| Light exposure | 7 days | 99.2 | No change |
| | 14 days | 98.9 | No change |
| | 1 month | 98.1 | No change |

**Table 33. Influencing factors of crystal form α of maleate**

| **Condition** | **Time** | **Purity (%)** | **Crystal form** |
|---|---|---|---|
| / | 0 days | 99.6 | Crystal form α of maleate |
| 40°C | 7 days | 98.9 | No change |
| | 14 days | 98.4 | No change |
| | 1 month | 97.4 | No change |
| 60°C | 7 days | 98.6 | No change |
| | 14 days | 97.0 | No change |
| | 1 month | 92.2 | No change |
| 75% RH | 7 days | 99.8 | No change |
| | 14 days | 99.8 | No change |
| | 1 month | 99.8 | No change |
| 92.5% RH | 7 days | 99.8 | No change |
| | 14 days | 99.8 | No change |
| | 1 month | 99.8 | No change |
| Light exposure | 7 days | 99.2 | No change |
| | 14 days | 98.7 | No change |
| | 1 month | 98.2 | No change |

**Table 34. Influencing factors of crystal form I of hydrobromide**

| **Condition** | **Time** | **Purity (%)** | **Crystal form** |
|---|---|---|---|
| / | 0 days | 99.7 | Crystal form I of hydrobromide |
| 40 °C | 7 days | 99.5 | No change |
| | 14 days | 99.3 | No change |
| | 1 month | 98.9 | No change |
| 60 °C | 7 days | 99.2 | No change |
| | 14 days | 99.0 | No change |
| | 1 month | 98.2 | No change |
| 75% RH | 7 days | 99.7 | No change |
| | 14 days | 99.6 | No change |
| | 1 month | 99.6 | No change |
| 92.5% RH | 7 days | 99.7 | No change |
| | 14 days | 99.6 | No change |
| | 1 month | 99.7 | No change |
| Light exposure | 7 days | 99.2 | No change |
| | 14 days | 99.1 | No change |
| | 1 month | 98.6 | No change |

**Table 35. Influencing factors of crystal form I of hydrochloride**

| **Condition** | **Time** | **Purity (%)** | **Crystal form** |
|---|---|---|---|
| / | 0 days | 99.8 | Crystal form I of hydrochloride |
| 40°C | 7 days | 99.3 | No change |
| | 14 days | 99.0 | No change |
| | 1 month | 99.0 | No change |
| 60°C | 7 days | 98.8 | No change |
| | 14 days | 97.8 | No change |
| | 1 month | 98.0 | No change |
| 75% RH | 7 days | 99.8 | No change |
| | 14 days | 99.6 | No change |
| | 1 month | 99.6 | No change |
| 92.5% RH | 7 days | 99.7 | No change |
| | 14 days | 99.6 | No change |
| | 1 month | 99.6 | No change |
| Light exposure | 7 days | 99.0 | No change |
| | 14 days | 98.7 | No change |
| | 1 month | 98.3 | No change |

Conclusion: The influencing factor experiments show that the crystal form b of tartrate and the crystal form α of maleate were slightly degraded at a high temperature, and the remaining salt forms exhibited good physical and chemical stability under the influencing factor conditions.

### Example 39: Long-Term/Accelerated Stability Study of Salt Forms

Samples of the crystal form I of mesylate, crystal form b of tartrate, crystal form a of sulfate, crystal form α of maleate, crystal form I of hydrobromide, and crystal form I of hydrochloride were sealed in aluminum foil bags and left to stand under conditions of 25 °C/60% RH and 40 °C/75% RH to study their stability.

**Table 36. Long-term accelerated stability of crystal form I of mesylate**

| **Condition** | **Time** | **Purity (%)** | **Crystal form** |
|---|---|---|---|
| / | 0 days | 99.8 | Crystal form I of mesylate |
| 25 °C-60% RH | 1 month | 99.8 | No change |
| | 2 months | 99.8 | No change |
| | 3 months | 99.8 | No change |
| | 6 months | 99.8 | No change |
| | 1 month | 99.8 | No change |
| 40 °C-75% RH | 2 months | 99.8 | No change |
| | 3 months | 99.8 | No change |
| | 6 months | 99.7 | No change |

**Table 37. Long-term accelerated stability of crystal form b of tartrate**

| **Condition** | **Time** | **Purity (%)** | **Crystal form** |
|---|---|---|---|
| / | 0 days | 99.8 | Crystal form b of tartrate |
| 25 °C-60% RH | 1 month | 99.7 | No change |
| | 2 months | 99.6 | No change |
| | 3 months | 99.5 | No change |
| | 6 months | 99.3 | No change |
| 40 °C-75% RH | 1 month | 99.1 | No change |
| | 2 months | 98.9 | No change |
| | 3 months | 98.7 | No change |
| | 6 months | 97.4 | No change |

**Table 38. Long-term accelerated stability of crystal form a of sulfate**

| **Condition** | **Time** | **Purity (%)** | **Crystal form** |
|---|---|---|---|
| / | 0 days | 99.7 | Crystal form a of sulfate |
| 25 °C-60% RH | 1 month | 99.7 | No change |
| | 2 months | 99.7 | No change |
| | 3 months | 99.8 | No change |
| | 6 months | 99.7 | No change |
| 40 °C-75% RH | 1 month | 99.6 | No change |
| | 2 months | 99.7 | No change |
| | 3 months | 99.6 | No change |
| | 6 months | 99.6 | No change |

**Table 39. Long-term accelerated stability of crystal form α of maleate**

| **Condition** | **Time** | **Purity (%)** | **Crystal form** |
|---|---|---|---|
| / | 0 days | 99.6 | Crystal form α of maleate |
| | 1 month | 99.8 | No change |
| 25 °C-60% RH | 2 months | 99.8 | No change |
| | 3 months | 99.8 | No change |
| | 6 months | 99.6 | No change |
| | 1 month | 99.6 | No change |
| 40 °C-75% RH | 2 months | 99.5 | No change |
| | 3 months | 99.3 | No change |
| | 6 months | 98.8 | No change |

**Table 40. Long-term accelerated stability of crystal form I of hydrobromide**

| **Condition** | **Time** | **Purity (%)** | **Crystal form** |
|---|---|---|---|
| / | 0 days | 99.7 | Crystal form I of hydrobromide |
| 25 °C-60% RH | 1 month | 99.8 | No change |
| | 2 months | 99.7 | No change |
| | 3 months | 99.7 | No change |
| | 6 months | 99.7 | No change |
| 40 °C-75% RH | 1 month | 99.6 | No change |
| | 2 months | 99.4 | No change |
| | 3 months | 97.9 | No change |
| | 6 months | 97.9 | No change |

**Table 41. Long-term accelerated stability of crystal form I of hydrochloride**

| **Condition** | **Time** | **Purity (%)** | **Crystal form** |
|---|---|---|---|
| / | 0 days | 99.8 | Crystal form I of hydrochloride |
| | 1 month | 99.3 | No change |
| 25 °C-60% RH | 2 months | 99.3 | No change |
| | 3 months | 99.3 | No change |
| | 6 months | 99.3 | No change |
| | 1 month | 99.2 | No change |
| 40 °C-75% RH | 2 months | 98.9 | No change |
| | 3 months | 98.8 | No change |
| | 6 months | 98.8 | No change |

The long-term/accelerated stability studies show that the crystal form I of mesylate, the crystal form a of sulfate, the crystal form α of maleate, and the crystal form I of hydrochloride exhibited good physical and chemical stability after storage under long-term accelerated conditions for 6 months; the crystal form I of hydrobromide was stable and the crystal form b of tartrate exhibited good stability under long-term conditions.

## Claims

1. A pharmaceutically acceptable salt of the compound of formula 1, (*S*)-4-(3-acetyl-5-ethoxy-2,8-dimethyl-1,4-dihydro-1,6-naphthyridin-4-yl)-3-(methoxy-*d₃*)benzonitrile, wherein the pharmaceutically acceptable salt is selected from the group consisting of hydrochloride, sulfate, phosphate, hydrobromide, mesylate, p-toluenesulfonate, tartrate, maleate, citrate, and malate,

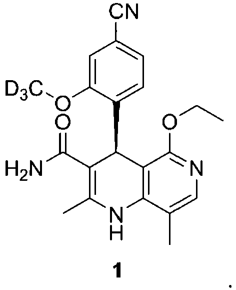

2. The pharmaceutically acceptable salt according to claim 1, wherein the chemical ratio of (*S*)-4-(3-acetyl-5-ethoxy-2,8-dimethyl-1,4-dihydro-1,6-naphthyridin-4-yl)-3-(methoxy-*d₃*)benzonitrile to an acid is 3:1-1:3, preferably 2:1-1:2, and more preferably 2:1 or 1:1.

3. A method for preparing the pharmaceutically acceptable salt according to claim 1, comprising:
reacting (*S*)-4-(3-acetyl-5-ethoxy-2,8-dimethyl-1,4-dihydro-1,6-naphthyridin-4-yl)-3-(methoxy-*d₃*)benzonitrile with an acid, wherein the acid is selected from the group consisting of hydrochloric acid, sulfuric acid, phosphoric acid, hydrobromic acid, methanesulfonic acid, p-toluenesulfonic acid, tartaric acid, maleic acid, citric acid, and malic acid.

4. A crystal form I of mesylate of the compound of formula 1, wherein an X-ray powder diffraction pattern expressed in terms of 2*θ* angles, which are diffraction angles, of the crystal form has characteristic peaks at 7.401, 8.635, 9.630, 17.457, and 26.350, preferably at 7.401, 8.635, 9.630, 14.895, 17.457, 19.014, 26.350, and 27.833, and more preferably at 7.401, 8.635, 9.630, 14.895, 15.590, 17.457, 19.014, 19.529, 22.735, 26.350, and 27.833.

5. The crystal form I of mesylate according to claim 4, wherein the X-ray powder diffraction pattern expressed in terms of 2*θ* angles, which are diffraction angles, of the crystal form is shown in FIG. 2.

6. A method for preparing the crystal form I of mesylate according to claim 4 or 5, comprising:
method I: dissolving the compound of formula 1 in 90% acetone/water, adding an aqueous methanesulfonic acid solution, and stirring;
method II: adding the compound of formula 1 to a solvent (1), adding a solution of methanesulfonic acid in ethanol, and slurrying for crystallization, wherein the solvent (1) is selected from the group consisting of isopropanol, isopropyl acetate, and 4-methyl-2-pentanone;
method III: dissolving the compound of formula 1 in acetonitrile/water (v/v = 1:1), adding a solution of methanesulfonic acid in ethanol, completely dissolving the mixture, and volatilizing for crystallization.

7. A crystal form α of maleate of the compound of formula 1, wherein an X-ray powder diffraction pattern expressed in terms of 2*θ* angles, which are diffraction angles, of the crystal form has characteristic peaks at 7.147, 8.562, 17.254, 20.869, and 26.061, preferably at 7.147, 8.562, 11.015, 12.787, 14.399, 17.254, 20.869, 22.478, and 26.061.

8. The crystal form α of maleate according to claim 7, wherein the X-ray powder diffraction pattern expressed in terms of 2*θ* angles, which are diffraction angles, of the crystal form is shown in FIG. 3.

9. A method for preparing the crystal form α of maleate according to claim 7 or 8, comprising:
method I: adding the compound of formula 1 to a solvent (2), adding maleic acid, and slurrying for crystallization, wherein the solvent (2) is selected from the group consisting of isopropanol, isopropyl acetate, 4-methyl-2-pentanone, and 90% acetonitrile/water;
method II: adding the compound of formula 1 to ethanol/dichloromethane (v/v = 1:1), adding an aqueous maleic acid solution, completely dissolving the mixture, and adding n-heptane for crystallization.

10. A crystal form a of sulfate of the compound of formula 1, wherein an X-ray powder diffraction pattern expressed in terms of 2*θ* angles, which are diffraction angles, of the crystal form has characteristic peaks at 7.130, 8.699, 17.528, 22.332, and 26.473, preferably at 7.130, 8.699, 9.465, 11.261, 17.528, 18.564, 22.332, and 26.473, and more preferably at 7.130, 8.699, 9.465, 11.261, 14.388, 15.667, 17.528, 18.564, 22.332, 22.798, 26.473, and 27.331.

11. The crystal form a of sulfate according to claim 10, wherein the X-ray powder diffraction pattern expressed in terms of 2*θ* angles, which are diffraction angles, of the crystal form is shown in FIG. 4.

12. A method for preparing the crystal form a of sulfate according to claim 10 or 11, comprising:
method I: dissolving the compound of formula 1 in 90% acetone/water, adding an aqueous sulfuric acid solution, and stirring;
method II: adding the compound of formula 1 to a solvent (3), adding a solution of sulfuric acid in ethanol, and slurrying for crystallization, wherein the solvent (3) is selected from the group consisting of isopropanol, isopropyl acetate, and 4-methyl-2-pentanone;
method III: dissolving the compound of formula 1 in acetonitrile/water (v/v = 1:1), adding a solution of sulfuric acid in ethanol, completely dissolving the mixture, and volatilizing for crystallization.

13. A crystal form b of tartrate of the compound of formula 1, wherein an X-ray powder diffraction pattern expressed in terms of 2*θ* angles, which are diffraction angles, of the crystal form has characteristic peaks at 8.674, 9.822, 11.826, 17.482, 22.566, and 26.431, preferably at 6.823, 7.456, 8.674, 9.822, 11.826, 17.482, 19.798, 22.566, and 26.431, and more preferably at 6.823, 7.456, 8.674, 9.822, 11.826, 17.482, 19.798, 22.566, 23.584, 25.602, 26.431, 27.215, and 27.755.

14. The crystal form b of tartrate according to claim 13, wherein the X-ray powder diffraction pattern expressed in terms of 2*θ* angles, which are diffraction angles, of the crystal form is shown in FIG. 6.

15. A method for preparing the crystal form b of tartrate according to claim 13 or 14, comprising:
method I: adding the compound of formula 1 to 90% acetonitrile/water, adding tartaric acid, and slurrying for crystallization;
method II: adding the compound of formula 1 to ethanol/dichloromethane (v/v = 1:1), adding an aqueous tartaric acid solution, completely dissolving the mixture, adding n-heptane, and slurrying for crystallization.

16. A crystal form A of the compound of formula 1, wherein an X-ray powder diffraction pattern expressed in terms of 2*θ* angles, which are diffraction angles, of the crystal form has characteristic peaks at 8.459, 13.964, 18.987, 22.914, and 25.530, preferably at 8.459, 13.964, 17.119, 18.987, 20.481, 22.914, 25.530, and 26.428, and more preferably at 8.459, 13.248, 13.964, 17.119, 18.987, 19.709, 20.481, 22.914, 23.874, 25.530, and 26.428.

17. The crystal form A according to claim 16, wherein the X-ray powder diffraction pattern expressed in terms of 2*θ* angles, which are diffraction angles, of the crystal form is shown in FIG. 16.

18. A method for preparing the crystal form A according to claim 16 or 17, being selected from the group consisting of:
method I: adding the compound of formula 1 to a solvent I, and slurrying for crystallization, wherein
the solvent I is selected from the group consisting of one or more of water, isopropanol, isopropyl acetate, methyl tert-butyl ether, isopropyl ether, toluene, n-heptane, ethyl acetate, 4-methyl-2-pentanone, and acetonitrile; and
method II: dissolving the compound of formula 1 in a solvent II, and adding a solvent III for crystallization, wherein the solvent II is selected from the group consisting of one or more of dimethyl sulfoxide, methanol, dichloromethane, acetonitrile, and acetone, and the solvent III is selected from the group consisting of one or more of water, isopropyl ether, isopropyl acetate, n-heptane, and methyl tert-butyl ether.

19. The crystal form according to any one of claims 4-5, 7-8, 10-11, 13-14, and 16-17, wherein the 2*θ* angles have a margin of error of ±0.20.

20. A pharmaceutical composition, comprising the following components:
i) the crystal form according to any one of claims 4-5, 7-8, 10-11, 13-14, and 16-17 or the pharmaceutically acceptable salt according to any one of claims 1-2; and
ii) one or more pharmaceutically acceptable excipients.

21. A method for preparing a pharmaceutical composition, comprising a step of mixing the crystal form according to any one of claims 4-5, 7-8, 10-11, 13-14, and 16-17 or the pharmaceutically acceptable salt according to any one of claims 1-2 with a pharmaceutically acceptable excipient.

22. Use of the crystal form according to any one of claims 4-5, 7-8, 10-11, 13-14, and 16-17, or the pharmaceutically acceptable salt according to any one of claims 1-2, or the pharmaceutical composition according to claim 20 in the manufacture of a medicament for treating and/or preventing a disease or disorder associated with a mineralocorticoid.

23. Use of the crystal form according to any one of claims 4-5, 7-8, 10-11, 13-14, and 16-17, or the pharmaceutically acceptable salt according to any one of claims 1-2, or the pharmaceutical composition according to claim 20 in the manufacture of a medicament for preventing and/or treating diabetic nephropathy, hyperaldosteronism, hypertension, and heart failure.
